# EUROPEAN PATENT APPLICATION

(11) **EP 4 406 961 A1**
(43) Date of publication of application: **31.07.2024**
(21) Application number: 22872987.7
(22) Date of filing: 22.09.2022
(51) Int. Cl.: C07K 5/02, C07K 5/08

(54) **PEPTIDE**

(30) Priority: 22.09.2021 JP 2021154625
(71) Applicant: AGC INC., Chiyoda-ku, Tokyo 1008405 (JP); The University of Tokyo, Bunkyo-ku, Tokyo 113-8654 (JP)
(72) Inventor: KASHIWAGI, Kimiaki, Tokyo 100-8405 (JP); OKAZOE, Takashi, Tokyo 100-8405 (JP); AIKAWA, Kohsuke, Tokyo 113-8654 (JP); SANDO, Shinsuke, Tokyo 113-8654 (JP); MORIMOTO, Jumpei, Tokyo 113-8654 (JP); KADOTA, Koji, Tokyo 113-8654 (JP); KOHATA, Ai, Tokyo 113-8654 (JP)
(74) Representative: Müller-Boré & Partner Patentanwälte PartG mbB
(86) International application number: PCT/JP2022/035392
(87) International publication number: WO 2023/048236

(57) **Abstract**

The present invention provides a peptide having a fluoroalkyl group in the side chain. The present invention provides a peptide in which 2 or more amino acids are peptide-bonded in which at least one side chain of an amino acid residue constituting the peptide is represented by the following General Formula (1) [in the formula, Z¹ is a divalent, trivalent, or tetravalent linking group other than an alkylene group; Rf is a C₁₋₃₀ alkyl group substituted with at least two fluorine atoms, -SF₅, or -SF₄-CR¹⁰¹R¹⁰²-CR¹⁰³R¹⁰⁴Cl (R¹⁰¹, R¹⁰², R¹⁰³, and R¹⁰⁴ are each independently a hydrogen atom, a fluorine atom, or a chlorine atom, and two or more of R¹⁰¹, R¹⁰², R¹⁰³, and R¹⁰⁴ are fluorine atoms); n3 is 1, 2, of 3, and the black circle indicates a bonding site].

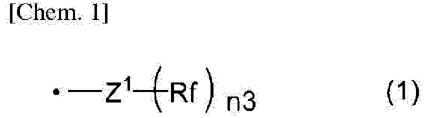

## Description

### [Technical Field]

The present invention relates to a peptide containing an amino acid residue with a fluorine atom introduced into a side chain.

Priority is claimed on Japanese Patent Application No. 2021-154625, filed September 22, 2021, the content of which is incorporated herein by reference.

### [Background Art]

Antibody drugs, peptide drugs, nucleic acid drugs and the like have advantages of having high specificity for target molecules and few side effects. However, all drugs have a problem in that it is difficult to reach target molecules inside cells. In order to solve this problem, various methods have been considered. Among these, cell-penetrating peptides (CPP) have been considered to be promising. Examples of typical CPPs include a peptide derived from TAT proteins of HIV viruses (Patent Document 1) and a poly-Arg sequence peptide (Patent Document 2). By combining these with a medicinal peptide, the medicinal peptide can be transported into cells (for example, Patent Document 3, and Non-Patent Document 1).

On the other hand, fluorine-containing amino acids have been reported to exhibit unique physiological activity, and are being focused on. For example, 3,3,3-trifluoroalanine and its derivatives have been reported to function as pyridoxal enzyme suicide inhibitors (Non-Patent Document 2). In addition, it has been reported that alanine racemase of the gram-negative bacteria *Salmonella typhimurium* and the gram-positive bacteria *Bacillus stearothermophilus* is inactivated with 3,3,3-trifluoroalanine (Non-Patent Document 3). Fluorine-containing amino acids and peptides containing them are expected to be used as physiologically active substances in the field of pharmaceuticals.

Compounds having a polyfluoro structure are known to be stable in living organisms, have low toxicity, and have excellent uptake into cells and escape from endosomes (Non-Patent Document 4). Based on these properties, it has been found that a peptide dendrimer using lysine whose side chain amino group is perfluoroacylated as a constituent amino acid can be used for gene delivery (Non-Patent Document 5). However, because the peptide is a dendrimer, it cannot form a hybrid that binds to a medicinally active peptide, a nucleic acid, or a protein that can be used as an antibody drug, like CPP.

### [Citation List]

### [Patent Document]

[Patent Document 1]
   United States Patent No. 6,316,003
[Patent Document 2]
   United States Patent No. 6,306,993
[Patent Document 3]
   PCT International Publication No. WO 2008/089491

### [Non-Patent Document]

[Non-Patent Document 1]
   Miyaji et. al., Drug Metabolism and Disposition, 2011, vol. 39, p. 1946-1953.
[Non-Patent Document 2]
   Sakai et al., Tetrahedron, 1996, vol. 52(1), p. 233-244.
[Non-Patent Document 3]
   Faraci and Walsh, Biochemistry, 1989, vol. 28(2), p. 431-437.
[Non-Patent Document 4]
   Zhang et al., MRS Communications, 2018, vol. 8, p. 303-313.
[Non-Patent Document 5]
   Cai et al., ACS Applied Materials and Interfaces, 2016, vol. 8, p. 5821-5832.

### [Summary of Invention]

### [Technical Problem]

CPPs described in Patent Document 1 and the like have various problems such as efficiency of transport into cells and degradation with peptidases in living organisms.

An object of the present invention is to provide a peptide containing an amino acid residue with a fluorine atom introduced into a side chain and a method of producing the same.

### [Solution to Problem]

The inventors found that, when a peptide containing an amino acid residue with a fluorine atom introduced into a side chain is produced, the peptide is highly effective at permeating a cell membrane, and thus completed the present invention.

Specifically, the present invention is as follows.
[1] A peptide in which 2 or more amino acids are peptide-bonded,
   wherein at least one side chain of an amino acid residue constituting the peptide is represented by the following General Formula (1): 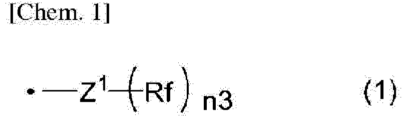 [in the formula, Z¹ is a divalent, trivalent, or tetravalent linking group other than an alkylene group; Rf is a C₁₋₃₀ alkyl group substituted with at least two fluorine atoms (the C₁₋₃₀ alkyl group may have 1 to 5 ether-bonding oxygen atoms between carbon atoms when the number of carbon atoms is 2 or more), -SF₅, or -SF₄-CR¹⁰¹R¹⁰²-CR¹⁰³R¹⁰⁴Cl (R¹⁰¹, R¹⁰², R¹⁰³, and R¹⁰⁴ are each independently a hydrogen atom, a fluorine atom, or a chlorine atom, and two or more of R¹⁰¹, R¹⁰², R¹⁰³, and R¹⁰⁴ are fluorine atoms); and n3 is 1, 2, or 3, and the black circle indicates a bonding site].
[2] The peptide according to [1],
   wherein Rf is a group represented by the following General Formula (f-1) or (f-2): 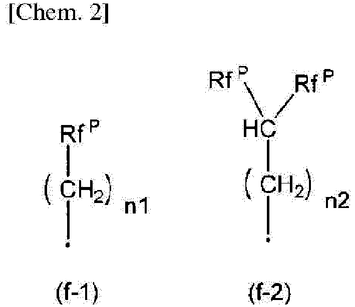 [in the formula, Rf^{P} represents a fully halogenated C₁₋₁₀ alkyl group including at least two fluorine atoms (the C₁₋₁₀ alkyl group may have an ether-bonding oxygen atom between carbon atoms when the number of carbon atoms is 2 or more), n1 is an integer of 0 to 10, n2 is an integer of 0 to 9, and the black circle indicates a bonding site].
[3] The peptide according to [1] or [2],
   wherein Z¹ is a linking group having a ring group.
[4] The peptide according to [3],
   wherein the ring group includes a 1,4-phenylene group or a 1,3,5-substituted phenyl group.
[5] The peptide according to [1] or [2],
   wherein Z¹ is a linking group having no ring group.
[6] The peptide according to [1] or [2],
   wherein Z¹ is an alkylene group, -O-, -S-, -NH-, -N(CH₃)-, -N(C₂H₅)-, -N(C₃H₇)-, a trivalent nitrogen atom, -C(=O)-, -S(=O)₂-, a group obtained by removing 2 to 4 hydrogen atoms from a cycloalkane, a group obtained by removing 2 to 4 hydrogen atoms from an aromatic ring, a group obtained by removing 2 to 4 hydrogen atoms from a heterocycle, or a combination thereof (excluding a group composed of only an alkylene group).
[7] The peptide according to [1] or [2],
   wherein Z¹ is -C(=O)-, -C(=O)-O-, -O-C(=O)-, -NH-C(=O)-O-, -O-C(=O)-IVH-, - C(=O)-NH-, -NH-C(=O)-, -S-S-, -S(=O)₂-NH-, -NH-S(=O)₂-, -S(=O)₂-NH-S(=O)₂-, - C(=O)-NH-Ph- (-Ph- is a 1,4-phenylene group, 1,3-phenylene group, 1,5-phenylene group, or 1,3,5-substituted phenyl group), or a combination of any of these groups and a C₁₋₆ alkylene group.
[8] The peptide according to [1] or [2],
   wherein Z¹ is a linking group represented by the following General Formula (2): 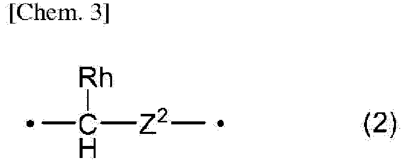 [in the formula, Z² is a divalent, trivalent, or tetravalent linking group other than an alkylene group; Rh is a hydrogen atom or a C₁₋₆ alkyl group, and the black circle indicates a bonding site].
[9] The peptide according to any one of [1] to [8],
   wherein the amino acid residue whose side chain is a group represented by General Formula (1) is an amino acid residue in which 1 to 3 Rfs are directly or indirectly linked to a side chain of a natural amino acid.
[10] The peptide according to any one of [1] to [9],
   wherein the C-terminal or N-terminal is optionally protected with a protecting group.
[11] The peptide according to [1] or [2], which is a tripeptide represented by the following General Formula (11): 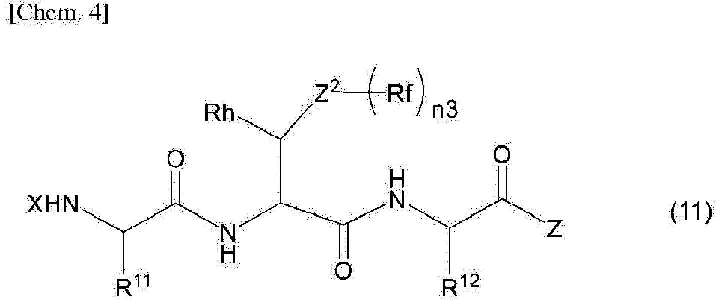 [in the formula, Z¹, Rf, and n3 are the same as above; Rh is a hydrogen atom or a C₁₋₆ alkyl group; R¹¹ and R¹² are each independently a C₁₋₆ alkyl group or a benzyl group; X is a 9-fluorenylmethyloxycarbonyl group or a tert-butoxycarbonyl group; and Z is a C₁₋₆ alkoxy group, a hydroxyl group, or an amino group].
[12] The peptide according to any one of [1] to [11], which has the ability to permeate a cell membrane.

### [Advantageous Effects of Invention]

Since the peptide according to the present invention has a fluorine atom introduced into the side chain, it is highly effective at permeating a cell membrane. Therefore, the peptide is expected to be used as a physiologically active substance in the field of pharmaceuticals.

### [Brief Description of Drawings]

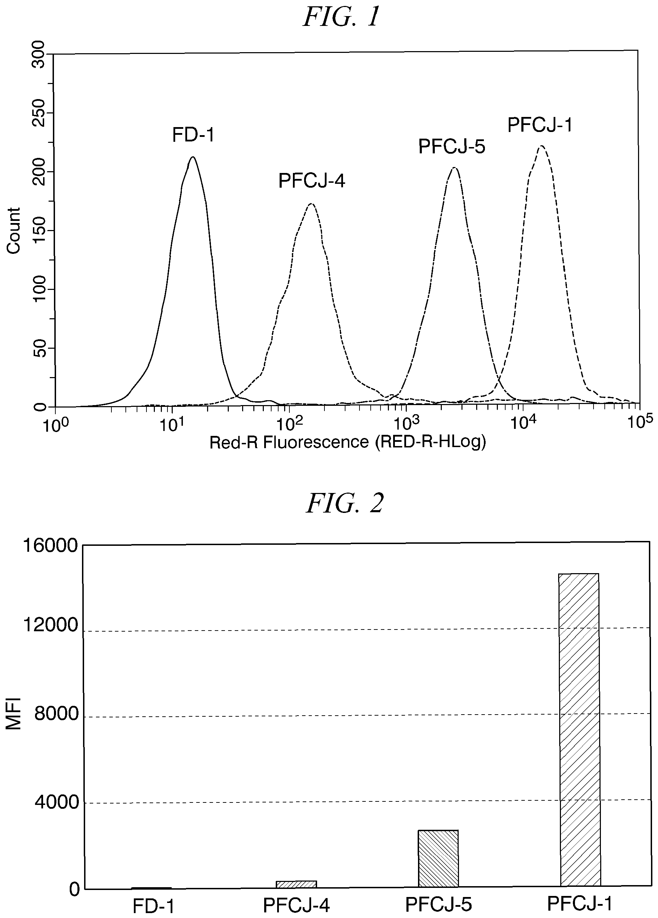

FIG. 1 is a diagram showing the flow cytometry results of HeLa cells treated in a sample solution containing a peptide fluorescent conjugate 1 (Alexa-Ala-Asp(C₈F₁₇)-Phe-NH₂) (PFCJ-1), a peptide fluorescent conjugate 4 (Alexa-Ala-[(R)-RFAA(C8)]-Phe-OMe) (PFCJ-4), a peptide fluorescent conjugate 5 (Alexa-Ala-[(S)-RFAA(C8)]-Phe-OMe) (PFCJ-5), or a fluorescent dye 1 (diethylamide form of fluorescent substance Alexa Fluoro 647) (FD-1) at 37°C for 1 hour in Test Example 1.
FIG. 2 is a diagram showing the measurement results of the mean fluorescence intensity (MFI) of HeLa cells treated in a sample solution containing a peptide fluorescent conjugate 1 (Alexa-Ala-Asp(C₈F₁₇)-Phe-NH₂) (PFCJ-1), a peptide fluorescent conjugate 4 (Alexa-Ala-[(R)-RFAA(C8)]-Phe-OMe) (PFCJ-4), a peptide fluorescent conjugate 5 (Alexa-Ala-[(S)-RFAA(C8)]-Phe-OMe) (PFCJ-5), or a fluorescent dye 1 (FD-1) at 37°C for 1 hour in Test Example 1.

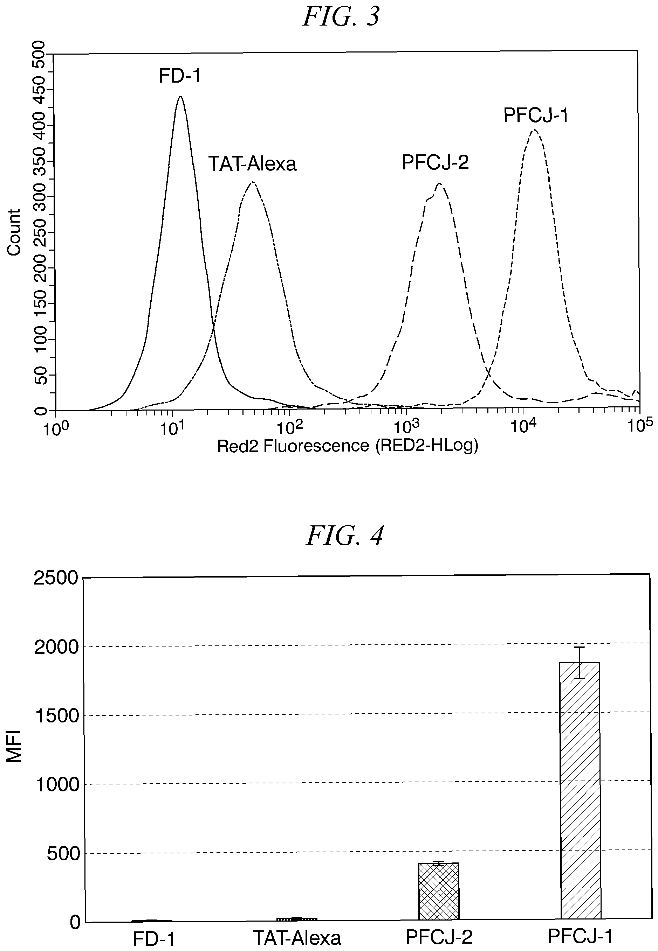

FIG. 3 is a diagram showing the flow cytometry results of HeLa cells treated in a sample solution containing a peptide fluorescent conjugate 1 (Alexa-Ala-Asp(C₈F₁₇)-Phe-NH₂) (PFCJ-1), a peptide fluorescent conjugate 2 (Alexa-Ala-Asp(bis-C₄F₉)-Phe-NH₂) (PFCJ-2), a fluorescent dye 1 (diethylamide form of fluorescent substance Alexa Fluoro 647) (FD1) or a fluorescent conjugate (TAT-Alexa) of a cell-penetrating peptide Cys-TAT(47-57) at 37°C for 1 hour in Test Example 2.
FIG. 4 is a diagram showing the measurement results of the mean fluorescence intensity (MFI) of HeLa cells treated in a sample solution containing a peptide fluorescent conjugate 1 (Alexa-Ala-Asp(C₈F₁₇)-Phe-NH₂) (PFCJ-1), a peptide fluorescent conjugate 2 (Alexa-Ala-Asp(bis-C₄F₉)-Phe-NH₂) (PFCJ-2), a fluorescent dye 1 (diethylamide form of fluorescent substance Alexa Fluoro 647) (FD-1), or a fluorescent conjugate (TAT-Alexa) of a cell-penetrating peptide Cys-TAT(47-57) at 37°C for 1 hour in Test Example 2.

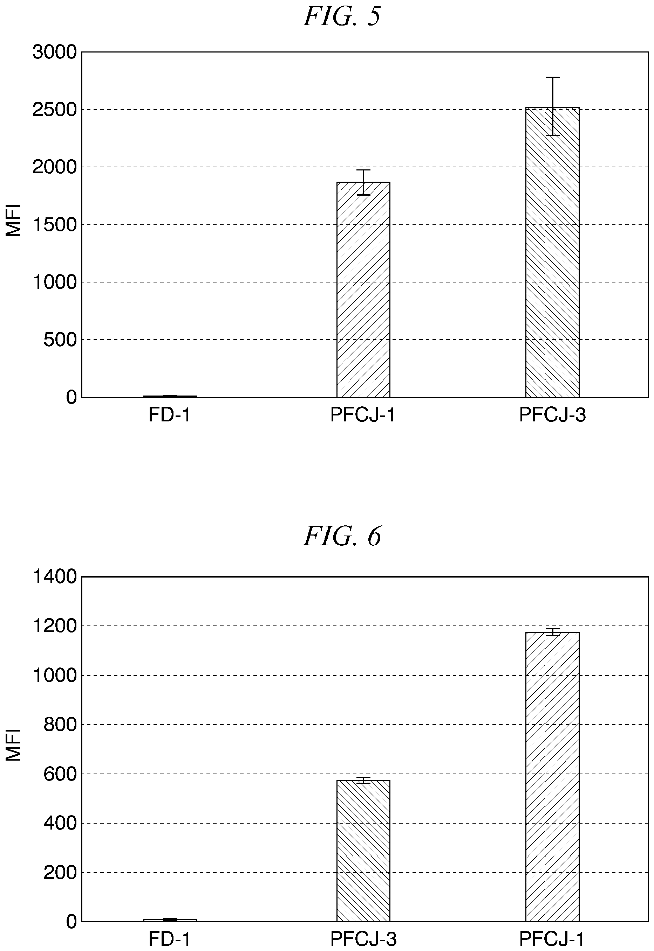

FIG. 5 is a diagram showing the measurement results of the mean fluorescence intensity (MFI) of HeLa cells treated in a sample solution containing a peptide fluorescent conjugate 1 (Alexa-Ala-Asp(C₈F₁₇)-Phe-NH₂) (PFCJ-1), a peptide fluorescent conjugate 3 (Alexa-Ala-Asp(C₁₂H₂₅)-Phe-NH₂) (PFCJ-3), or a fluorescent dye 1 (FD-1) in the absence of FBS at 37°C for 1 hour in Test Example 3.
FIG. 6 is a diagram showing the measurement results of the mean fluorescence intensity (MFI) of HeLa cells treated in a sample solution containing a peptide fluorescent conjugate 1 (Alexa-Ala-Asp(C₈F₁₇)-Phe-NH₂) (PFCJ-1), a peptide fluorescent conjugate 3 (Alexa-Ala-Asp(C₁₂H₂₅)-Phe-NH₂) (PFCJ-3), or a fluorescent dye 1 (FD-1) in the presence of FBS at 37°C for 1 hour in Test Example 3.

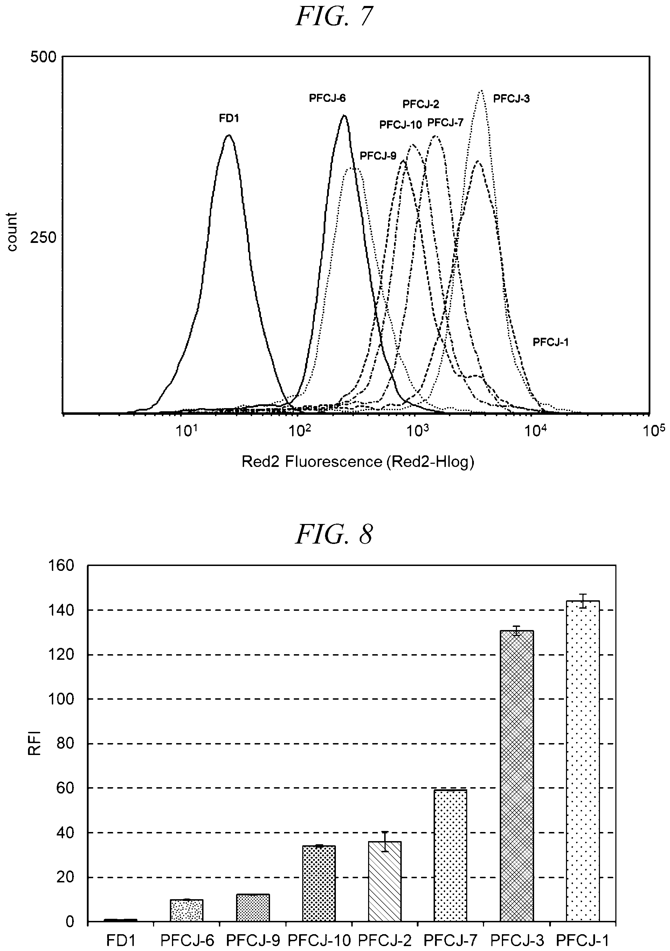

FIG. 7 is a diagram showing the flow cytometry results of HeLa cells treated in a sample solution containing a peptide fluorescent conjugate 1 (Alexa-Ala-Asp(C₈F₁₇)-Phe-NH₂) (PFCJ-1), a peptide fluorescent conjugate 2 (Alexa-Ala-Asp(bis-C₄F₉)-Phe-NH₂) (PFCJ-2), a peptide fluorescent conjugate 3 (Alexa-Ala-Asp(C₁₂H₂₅)-Phe-NH₂) (PFCJ-3), a peptide fluorescent conjugate 6 (Alexa-Ala-Asp(C₄F₉)-Phe-NH₂) (PFCJ-6), a peptide fluorescent conjugate 7 (Alexa-Ala-Asp(C₆F₁₃)-Phe-NH₂) (PFCJ-7), a peptide fluorescent conjugate 9 (Alexa-Ala-Asp(SF₄CF₂CF₂Cl)-Phe-NH₂) (PFCJ-9), a peptide fluorescent conjugate 10 (Alexa-Ala-Asp(tris-t-C₄F₉)-Phe-NH₂) (PFCJ-10), or a fluorescent dye 1 (FD-1) at 37°C for 1 hour in Test Example 4.
FIG. 8 is a diagram showing the measurement results of the relative fluorescence intensity (RFI) of each sample based on a fluorescent dye 1 of HeLa cells treated in a sample solution containing a peptide fluorescent conjugate 1 (Alexa-Ala-Asp(C₈F₁₇)-Phe-NH₂) (PFCJ-1), a peptide fluorescent conjugate 2 (Alexa-Ala-Asp(bis-C₄F₉)-Phe-NH₂) (PFCJ-2), a peptide fluorescent conjugate 3 (Alexa-Ala-Asp(C₁₂H₂₅)-Phe-NH₂) (PFCJ-3), a peptide fluorescent conjugate 6 (Alexa-Ala-Asp(C₄F₉)-Phe-NH₂) (PFCJ-6), a peptide fluorescent conjugate 7 (Alexa-Ala-Asp(C₆F₁₃)-Phe-NH₂) (PFCJ-7), a peptide fluorescent conjugate 9 (Alexa-Ala-Asp(SF₄CF₂CF₂Cl)-Phe-NH₂) (PFCJ-9), a peptide fluorescent conjugate 10 (Alexa-Ala-Asp(tris-t-C₄F₉)-Phe-NH₂) (PFCJ-10), or a fluorescent dye 1 (FD-1) at 37°C for 1 hour in Test Example 4.

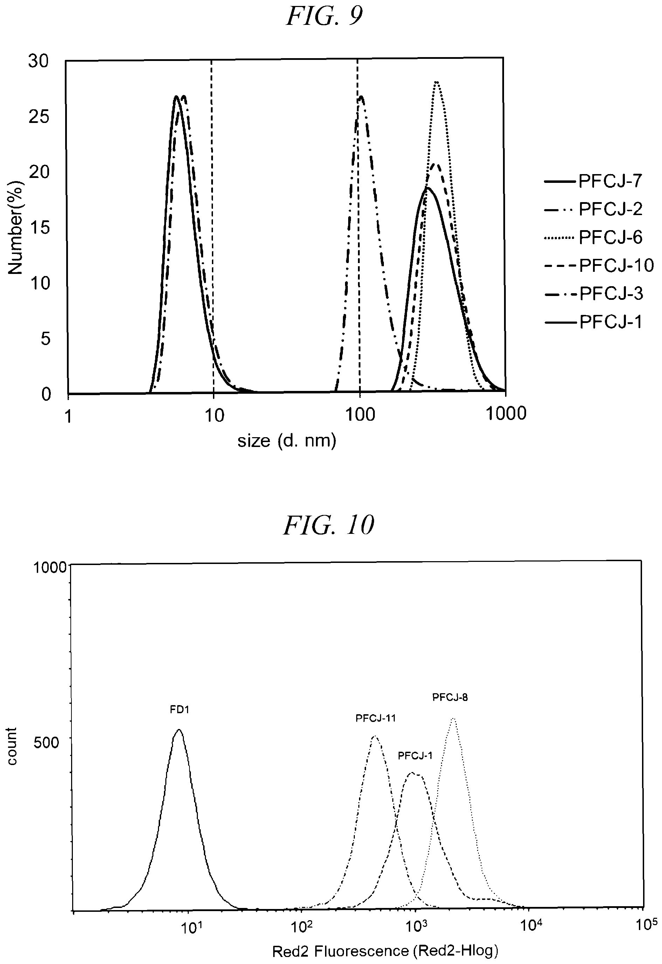

FIG. 9 is a diagram showing the measurement results of average particle sizes of a peptide fluorescent conjugate 1 (Alexa-Ala-Asp(C₈F₁₇)-Phe-NH₂) (PFCJ-1), a peptide fluorescent conjugate 2 (Alexa-Ala-Asp(bis-C₄F₉)-Phe-NH₂) (PFCJ-2), a peptide fluorescent conjugate 3 (Alexa-Ala-Asp(C₁₂H₂₅)-Phe-NH₂) (PFCJ-3), a peptide fluorescent conjugate 6 (Alexa-Ala-Asp(C₄F₉)-Phe-NH₂) (PFCJ-6), a peptide fluorescent conjugate 7 (Alexa-Ala-Asp(C₆F₁₃)-Phe-NH₂) (PFCJ-7), a peptide fluorescent conjugate 9 (Alexa-Ala-Asp(SF₄CF₂CF₂Cl)-Phe-NH₂) (PFCJ-9), and a peptide fluorescent conjugate 10 (Alexa-Ala-Asp(tris-t-C₄F₉)-Phe-NH₂) (PFCJ-10) in Test Example 5.
FIG. 10 is a diagram showing the flow cytometry results of HeLa cells treated in a sample solution containing a peptide fluorescent conjugate 1 (Alexa-Ala-Asp(C₈F₁₇)-Phe-NH₂) (PFCJ-1), a peptide fluorescent conjugate 8 (Alexa-Ala-Asp(C₁₀F₂₁)-Phe-NH₂) (PFCJ-8), a peptide fluorescent conjugate 11 (Alexa-Ala-Asp(CH₂CH₂C₈F₁₇)-Phe-NH₂) (PFCJ-11), or a fluorescent dye 1 (diethylamide form of fluorescent substance Alexa Fluoro 647) (FD-1) at 37°C for 1 hour in Test Example 6.

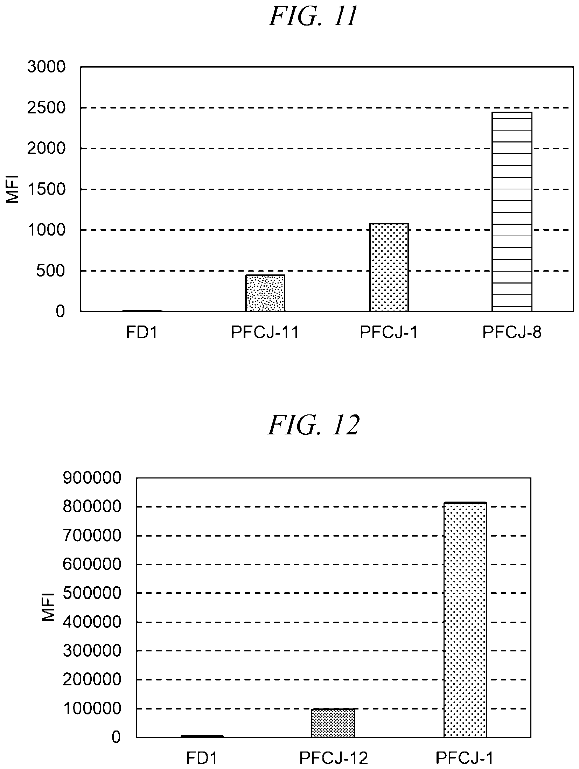

FIG. 11 is a diagram showing the measurement results of the mean fluorescence intensity (MFI) of HeLa cells treated in a sample solution containing a peptide fluorescent conjugate 1 (Alexa-Ala-Asp(C₈F₁₇)-Phe-NH₂) (PFCJ-1), a peptide fluorescent conjugate 8 (Alexa-Ala-Asp(C₁₀F₂₁)-Phe-NH₂) (PFCJ-8), a peptide fluorescent conjugate 11 (Alexa-Ala-Asp(CH₂CH₂C₈F₁₇)-Phe-NH₂) (PFCJ-11), or a fluorescent dye 1 (FD-1) at 37°C for 1 hour in Test Example 6.
FIG. 12 is a diagram showing the measurement results of the mean fluorescence intensity (MFI) of HeLa cells treated in a sample solution containing a peptide fluorescent conjugate 1 (Alexa-Ala-Asp(C₈F₁₇)-Phe-NH₂) (PFCJ-1), a peptide fluorescent conjugate 12 (Alexa-Ala-Asp(CH₂CH₂C₆F₁₃)-Phe-NH₂) (PFCJ-12), or a fluorescent dye 1 (FD-1) at 37°C for 1 hour in Test Example 7.

### [Description of Embodiments]

In the present invention and this specification, "Cₚ₁₋ₚ₂" (p1 and p2 are positive integers that satisfy p1<p2) indicates a group having at least p1 and no more than p2 carbon atoms.

In the present invention and this specification, the "C₁₋₃₀ alkyl group" is an alkyl group having 1 to 30 carbon atoms, and may be linear or branched. The "C₂₋₃₀ alkyl group" is an alkyl group having 2 to 30 carbon atoms, and may be linear or branched. Examples of C₁₋₃₀ alkyl groups include a methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, eicosyl group, henicosyl group, docosyl group, tricosyl group, tetracosyl group, pentacosyl group, hexacosyl group, heptacosyl group, octacosyl group, nonacosyl group, and triacontyl group.

In the present invention and this specification, the "C₁₋₁₀ alkyl group" is an alkyl group having 1 to 10 carbon atoms, and may be linear or branched. The "C₂₋₁₀ alkyl group" is an alkyl group having 2 to 10 carbon atoms, and may be linear or branched. Examples of C₁₋₁₀ alkyl groups include a methyl group, ethyl group, propyl group, isopropyl group, butyl group, isobutyl group, sec-butyl group, tert-butyl group, pentyl group, isopentyl group, neopentyl group, tert-pentyl group, hexyl group, heptyl group, octyl group, nonyl group, and decyl group.

In the present invention and this specification, the "C₁₋₆ alkyl group" is an alkyl group having 1 to 6 carbon atoms, and may be linear or branched. Examples of C₁₋₆ alkyl groups include a methyl group, ethyl group, propyl group, isopropyl group, butyl group, isobutyl group, sec-butyl group, tert-butyl group, pentyl group, isopentyl group, neopentyl group, tert-pentyl group, and hexyl group.

In the present invention and this specification, the "C₆₋₁₄ aryl group" is an aromatic hydrocarbon group having 6 to 14 carbon atoms, and a C₆₋₁₂ aryl group is particularly preferable. Examples of C₆₋₁₄ aryl groups include a phenyl group, naphthyl group, anthryl group, and 9-fluorenyl group, and a phenyl group is particularly preferable.

In the present invention and this specification, the "optionally substituted C₆₋₁₄ aryl group" is a group in which one or more hydrogen atoms, preferably 1 to 3 hydrogen atoms bonded to carbon atoms of a C₆₋₁₄ aryl group, are substituted with other functional groups. When there are two or more substituents, the substituents may be of the same or different types. Examples of substituents include a nitro group, a halogen atom (a fluorine atom, a chlorine atom, a bromine atom, or an iodine atom), a C₁₋₆ alkyl group, a C₁₋₆ alkoxy group, and a methylenedioxy group (-O-CH₂-O-). Examples of "optionally substituted C₆₋₁₄ aryl groups" include a phenyl group, naphthyl group, anthryl group, 4-nitrophenyl group, 4-methoxyphenyl group, 2,4-dimethoxyphenyl group, 3,4-dimethoxyphenyl group, 4-methylphenyl group, 2,6-dimethylphenyl group, 3-chlorophenyl group, and 1,3-benzodioxol-5-yl group.

In the present invention and this specification, the "C₆₋₁₄ aryl-C₁₋₆ alkyl group" is a group in which one hydrogen atom bonded to a carbon atom of a C₁₋₆ alkyl group is substituted with a C₆₋₁₄ aryl group. Examples of C₆₋₁₄ aryl groups in the C₆₋₁₄ aryl-C₁₋₆ alkyl group include a phenyl group, naphthyl group, anthryl group, and 9-fluorenyl group, and a phenyl group or a 9-fluorenyl group is particularly preferable. The C₁₋₆ alkyl group in the C₆₋₁₄ aryl-C₁₋₆ alkyl group is preferably a C₁₋₄ alkyl group. Examples of C₆₋₁₄ aryl-C₁₋₆ alkyl groups include a benzyl group, diphenylmethyl group, triphenylmethyl group, 2-phenylethyl group, 9-anthrylmethyl group, and 9-fluorenylmethyl group.

In the present invention and this specification, the "halogen atom" refers to a fluorine atom, a chlorine atom, a bromine atom, or an iodine atom. The "halogen atom other than a fluorine atom" is a chlorine atom, a bromine atom, or an iodine atom. As an example of "a halogen atom other than a fluorine atom," a chlorine atom or a bromine atom is preferable, and a chlorine atom is particularly preferable.

In the present invention and this specification, the "C₁₋₆ alkoxy group" is a group in which an oxygen atom is bonded to the bond terminal of a C₁₋₆ alkyl group having 1 to 6 carbon atoms. The C₁₋₆ alkoxy group may be linear or branched. Examples of C₁₋₆ alkoxy groups include a methoxy group, ethoxy group, propoxy group, butoxy group, tert-butoxy group, pentyloxy group, and hexyloxy group.

In the present invention and this specification, the "ether-bonding oxygen atom" is an oxygen atom that links carbon atoms, and does not include oxygen atoms in which oxygen atoms are linked in series. The maximum number of ether-bonding oxygen atoms that an alkyl group having a carbon number Nc (Nc is an integer of 2 or more) can have is Nc-1.

In addition, hereinafter, "compound n" is a compound represented by Formula (n).

### <Fluorine-containing peptide>

The peptide according to the present invention is a peptide composed of 2 or more amino acids, in which at least one side chain of an amino acid residue constituting the peptide is a group represented by the following General Formula (1). In General Formula (1), the black circle indicates a bonding site. In addition, hereinafter, an amino acid in which a group represented by the following General Formula (1) is bonded to the α carbon will be referred to as a "fluorine-containing amino acid," and a peptide in which at least one side chain of an amino acid residue constituting a peptide is a group represented by the following General Formula (1) will be referred to as a "fluorine-containing peptide."

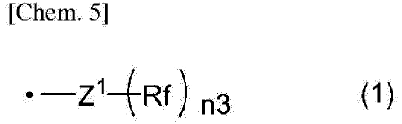

In General Formula (1), Rf is a C₁₋₃₀ alkyl group substituted with at least two fluorine atoms, -SF₅, or -SF₄-CR¹⁰¹R¹⁰²-CR¹⁰³R¹⁰⁴Cl.

When Rf is a C₁₋₃₀ alkyl group substituted with at least two fluorine atoms, the C₁₋₃₀ alkyl group may have 1 to 5 ether-bonding oxygen atoms between carbon atoms if the number of carbon atoms is 2 or more (in the case of a C₂₋₃₀ alkyl group).

In Rf, one or more hydrogen atoms bonded to a carbon atom may be additionally optionally substituted with a halogen atom other than a fluorine atom. Here, the C₁₋₃₀ alkyl group for Rf is preferably a C₁₋₂₀ alkyl group, more preferably a C₁₋₁₀ alkyl group, still more preferably a C₂₋₁₀ alkyl group, and yet more preferably a C₂₋₈ alkyl group. When the C1-30 alkyl group is a C₂₋₃₀ alkyl group, it may have 1 to 5 ether-bonding oxygen atoms between carbon atoms. In Rf, the number of hydrogen atoms substituted with fluorine atoms is not particularly limited as long as it is 2 or more, and for example, the number is preferably 3 or more, more preferably 6 or more, and still more preferably 7 or more.

Examples of Rf include a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group, perfluorodecyl group, difluoromethyl group, 1,1-difluoroethyl group, 2,2-difluoroethyl group, 1,1,2,2-tetrafluoroethyl group, 1,1,2,2,3,3-hexafluoropropyl group, 1,1,2,3,3,3-hexafluoropropyl group, 1,1,2,2,3,3-hexafluorohexyl group, 1,1,2,2,3,3-hexafluorooctyl group, 1,1,2,2,3,3-hexafluorodecyl group, 1,1,2,2,3,3-hexafluorooctadecyl group, and 1,1,2,2,3,3-hexafluorohexacosyl group.

When Rf is a group having 2 carbon atoms, Rf is preferably a group in which at least 4 or more hydrogen atoms bonded to carbon atoms are substituted with fluorine atoms, such as a pentafluoroethyl group rather than a 1,1,1-trifluoroethyl group (CF₃-CH₂-). In addition, when Rf is a group having 3 carbon atoms, Rf is preferably a linear group, and in the case of a branched group, a 1,1,1,3,3,3-hexafluoropropan-2-yl group ((CF₃)₂-CH-) or (CF₃)₂-CF-group is preferable. When Rf is a group having 4 carbon atoms, Rf may be a linear group or a branched group. In the case of a branched group, a group in which a hydrogen atom bonded to a carbon atom constituting the alkylene group moiety is substituted with a fluorine atom or a completely fluorinated group is preferable.

Rf is preferably a group represented by the following General Formula (f-1) or (f-2). Here, Rf^{P} represents a fully halogenated C₁₋₁₀ alkyl group including at least two fluorine atoms. Rf^{P} is a group in which all hydrogen atoms of a C₁₋₁₀ alkyl group are substituted with halogen atoms, and at least two or more of these halogen atoms are fluorine atoms. When Rf^{P} has 2 or more carbon atoms, that is, when it is a fully halogenated C₂₋₁₀ alkyl group, it may have 1 to 5 ether-bonding oxygen atoms between carbon atoms. In General Formula (f-2), two Rf^{P}'s may be the same group or different groups.

In the following General Formula (f-1) or (f-2), n1 is an integer of 0 to 10, and n2 is an integer of 0 to 9. When n1 and n2 are 0, both represent a single bond. That is, when n1 is 0, the group represented by General Formula (f-1) is Rf^{P}-, and when n2 is 0, the group represented by General Formula (f-2) is (Rf^{P})₂-CH-.

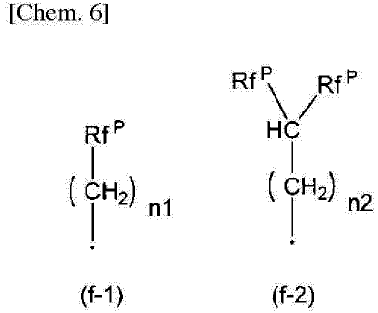

When Rf is a group represented by General Formula (f-1), Rf is preferably a group in which Rf^{P} is a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group, or perfluorodecyl group and n1 is an integer of 0 to 4, more preferably a group in which Rf^{P} is a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group, or perfluorodecyl group, and n1 is an integer of 0 to 2, and still more preferably a group in which Rf^{P} is a trifluoromethyl group, pentafluoroethyl group, heptafluoropropyl group, nonafluorobutyl group, perfluoropentyl group, or perfluorohexyl group, and n1 is an integer of 0 to 2 (excluding a group in which n1 is 1, and Rf^{P} is a trifluoromethyl group).

When Rf is a group represented by General Formula (f-2), Rf is preferably a group in which Rf^{P} is a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group, or perfluorodecyl group, and n2 is an integer of 0 to 4, more preferably a group in which Rf^{P} is a trifluoromethyl group, pentafluoroethyl group, heptafluoropropyl group, nonafluorobutyl group, perfluoropentyl group, perfluorohexyl group, perfluoroheptyl group, perfluorooctyl group, perfluorononyl group, or perfluorodecyl group, and n2 is an integer of 0 to 2, and still more preferably a group in which Rf^{P} is a trifluoromethyl group, pentafluoroethyl group, heptafluoropropyl group, nonafluorobutyl group, perfluoropentyl group, or perfluorohexyl group, and n2 is an integer of 0 to 2 (excluding a group in which n2 is 0 or 1 and Rf^{P} is a trifluoromethyl group).

Examples of Rf include a difluoromethyl group, 1,1-difluoroethyl group, 2,2-difluoroethyl group, 1,1,2,2-tetrafluoroethyl group, 1,1,2,2,3,3-hexafluoropropyl group, and 1,1,2,3,3,3-hexafluoropropyl group.

When Rf is a group represented by -SF₄-CR¹⁰¹R¹⁰²-CR¹⁰³R¹⁰⁴Cl, R¹⁰¹, R¹⁰², R¹⁰³, and R¹⁰⁴ are each independently a hydrogen atom, a fluorine atom, or a chlorine atom. Here, two or more of R¹⁰¹, R¹⁰², R¹⁰³, and R¹⁰⁴ are fluorine atoms. Specific examples of groups represented by -SF₄-CR¹⁰¹R¹⁰²-CR¹⁰³R¹⁰⁴Cl include -SF₄-CF₂-CF₂Cl, -SF₄-CF₂-CFCl₂, -SF₄-CF₂-CHF-Cl, -SF₄-CF₂-CCl₃, -SF₄-CF₂-CHCl₂, -SF₄-CF₂-CH₂Cl, -SF₄-CFCl-CFCl₂, -SF₄-CFCl-CHF-Cl, and -SF₄-CHF-CHF-Cl.

In General Formula (1), Z¹ is a divalent, trivalent, or tetravalent linking group other than an alkylene group, and n3 is 1, 2, or 3. Z¹ is not particularly limited as long as it is a divalent to tetravalent group other than an alkylene group. Examples of Z¹ include an alkylene group, an oxygen atom (-O-), a sulfur atom (-S-), -NH-, -N(CH₃)-, -N(C₂H₅)-, -N(C₃H₇)-, a trivalent nitrogen atom, -C(=O)-, -S(=O)₂-, a group obtained by removing 2 to 4 hydrogen atoms from a cycloalkane, a group obtained by removing 2 to 4 hydrogen atoms from an aromatic ring, a group obtained by removing 2 to 4 hydrogen atoms from a heterocycle, and a combination thereof. As the aryl group and the heteroaryl group, the groups listed above can be used. Here, a group composed of only an alkylene group and a group whose linking moiety with Rf is an alkylene group are excluded.

When Z¹ is an alkylene group, an oxygen atom (-O-), a sulfur atom (-S-), -NH-, - N(CH₃)-, -N(C₂H₅)-, -N(C₃H₇)-, -C(=O)-, -S(=O)₂-, a group obtained by removing two hydrogen atoms from a cycloalkane, a group obtained by removing two hydrogen atoms from an aromatic ring, a group obtained by removing two hydrogen atoms from a heterocycle or a combination thereof, Z¹ is a divalent linking group, and the group represented by General Formula (1) is a group having one Rf group. When Z¹ has a trivalent nitrogen atom, two Rf groups are directly bonded to the nitrogen atom or via another divalent linking group, and thus the group represented by General Formula (1) can be a group having two Rf groups.

Z¹ may be a linking group having a group (ring group) obtained by removing a hydrogen atom from a ring or may be a linking group having no ring group. When Z¹ has a ring group, the group represented by General Formula (1) can be a group having 2 or 3 Rf groups. Examples of ring groups include a group obtained by removing 2 to 4 hydrogen atoms from a cycloalkane, aromatic ring, or heterocycle. The heterocycle is preferably a ring in which 1 to 3 carbon atoms of an aromatic ring are substituted with one or more atoms selected from the group consisting of nitrogen atoms, oxygen atoms, and sulfur atoms. In addition, the ring group may be a group obtained by removing a hydrogen atom from a monocyclic ring or a group obtained by removing a hydrogen atom from a condensed ring. The ring group contained in Z¹ in General Formula (1) is preferably a group obtained by removing 2 to 4 hydrogen atoms from cyclohexane, benzene, imidazole, or indole. For example, when Z¹ is a linking group having a ring group obtained by removing 2 to 4 hydrogen atoms from benzene, the ring group is a 1,4-phenylene group, 1,3-phenylene group, 1,5-phenylene group, or 1,3,5-substituted phenyl group, and preferably a 1,4-phenylene group or 1,3,5-substituted phenyl group.

Specific examples of Z¹ in General Formula (1) include -C(=O)-, -C(=O)-O-, -O-C(=O)-, -NH-C(=O)-O-, -O-C(=O)-NH-, -C(=O)-NH-, -NH-C(=O)-, -S-S-, -S(=O)₂-NH-, -NH-S(=O)₂-, -S(=O)₂-NH-S(=O)₂-, and -C(=O)-NH-Ph- (-Ph- is a 1,4-phenylene group, 1,3-phenylene group, 1,5-phenylene group, or 1,3,5-substituted phenyl group). In addition, the linking group is preferably a combination of any of these groups and a C₁₋₆ alkylene group. Z¹ in General Formula (1) is preferably one in which the linking moiety with Rf is an oxygen atom.

Z¹ in General Formula (1) is preferably a linking group represented by the following General Formula (2).

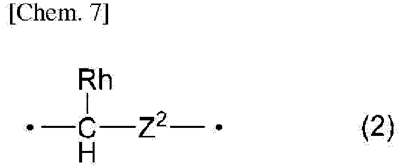

In General Formula (2), Z² is a divalent, trivalent, or tetravalent linking group other than an alkylene group. Z² is not particularly limited as long as it is a divalent to tetravalent group other than an alkylene group. Examples of Z² include an alkylene group, an oxygen atom (-O-), a sulfur atom (-S-), -NH-, -N(CH₃)-, -N(C₂H₅)-, -N(C₃H₇)-, a trivalent nitrogen atom, -C(=O)-, -S(=O)₂-, a group obtained by removing 2 to 4 hydrogen atoms from a cycloalkane, a group obtained by removing 2 to 4 hydrogen atoms from an aromatic ring, a group obtained by removing 2 to 4 hydrogen atoms from a heterocycle, and a combination thereof. As the aryl group and the heteroaryl group, the groups listed above can be used. Here, a group composed of only an alkylene group and a group whose linking moiety with Rf is an alkylene group are excluded.

As Z² in General Formula (2), specifically, the same ones as those listed for Z¹ can be used. Z² in General Formula (2) is preferably -C(=O)-, -C(=O)-O-, -O-C(=O)-, - NH-C(=O)-O-, -O-C(=O)-NH-, -C(=O)-NH-, -NH-C(=O)-, -S-S-, -S(=O)₂-NH-, -NH-S(=O)₂-, -S(=O)₂-NH-S(=O)₂-, or -C(=O)-NH-Ph- (-Ph- is a 1,4-phenylene group, 1,3-phenylene group, 1,5-phenylene group, or 1,3,5-substituted phenyl group).

In General Formula (2), Rh is a hydrogen atom or a C₁₋₆ alkyl group. When Rh is a C₁₋₆ alkyl group, Rh is preferably a C₁₋₃ alkyl group and more preferably a methyl group or an ethyl group.

As the group represented by General Formula (1), for example, a group in which Z¹ is a group represented by General Formula (2) and Rf is a group represented by General Formula (f-1) or (f-2) may be exemplified. Among these, a group in which Z² in General Formula (2) is -C(=O)-, -C(=O)-O-, -O-C(=O)-, -NH-C(=O)-O-, -O-C(=O)-NH-, -C(=O)-NH-, -NH-C(=O)-, -S-S-, -S(=O)₂-NH-, -NH-S(=O)₂-, -S(=O)₂-NH-S(=O)₂-, or -C(=O)-NH-Ph- (-Ph- is a 1,4-phenylene group, 1,3-phenylene group, 1,5-phenylene group, or 1,3,5-substituted phenyl group), Rh is a hydrogen atom or a C₁₋₆ alkyl group, and Rf is a group represented by General Formula (f-1) or (f-2) is preferable.

The fluorine-containing peptide according to the present invention is preferably a peptide in which an amino acid residue whose side chain is a group (-Z¹-(Rf)n3) represented by General Formula (1) is an amino acid residue in which 1 to 3 Rf groups are directly or indirectly linked to the side chain of a natural amino acid. Specifically, the fluorine-containing peptide according to the present invention is preferably a peptide having at least one amino acid residue in which 1 or 2 hydrogen atoms of an amino group in the side chain of an arginine residue, an asparagine residue, a glutamine residue, or a lysine residue is substituted with -Rf or -Z³-(Rf)n4 (Z³ is a divalent, trivalent, or tetravalent linking group other than an alkylene group, here, a group in which a linking moiety with Rf is an alkylene group is excluded, and n4 is 2, 3, or 4); amino acid residue in which a hydrogen atom of an imino group in the side chain of an arginine residue is substituted with Rf or -Z³-(Rf)n4; amino acid residue in which a hydrogen atom of a carboxyl group in the side chain of an asparagine acid residue or a glutamine acid residue is substituted with -Rf or -Z³-(Rf)n4; amino acid residue in which a hydrogen atom of a thiol group in the side chain of a cysteine residue or a methionine residue is substituted with -Rf or -Z³-(Rf)n4; amino acid residue in which a hydrogen atom of a hydroxy group in the side chain of a serine residue, a threonine residue, or a tryptophan residue is substituted with Rf or - Z³-(Rf)n4; amino acid residue in which 1 to 3 hydrogen atoms of a benzene ring in the side chain of a tyrosine residue or a phenylalanine residue is substituted with -Rf or -Z³-(Rf)n4; amino acid residue in which 1 to 3 hydrogen atoms of an imidazole ring in the side chain of a histidine residue is substituted with -Rf or -Z³-(Rf)n4; or amino acid residue in which 1 to 3 hydrogen atoms of an indole ring in the side chain of a tryptophan residue is substituted with -Rf or -Z³-(Rf)n4.

Z³ is not particularly limited as long as it is a divalent to tetravalent group other than an alkylene group. Examples of Z³ include an alkylene group, an oxygen atom (-O-), a sulfur atom (-S-), -NH-, -N(CH₃)-, -N(C₂H₅)-, -N(C₃H₇)-, a trivalent nitrogen atom, -C(=O)-, -S(=O)₂-, a group obtained by removing 2 to 4 hydrogen atoms from a cycloalkane, a group obtained by removing 2 to 4 hydrogen atoms from an aromatic ring, a group obtained by removing 2 to 4 hydrogen atoms from a heterocycle, and a combination thereof. As the aryl group and the heteroaryl group, the groups listed above can be used. Here, a group composed of only an alkylene group and a group whose linking moiety with Rf is an alkylene group are excluded. Specifically, as Z³, the same linking groups as in Z¹ and Z² can be used.

Substitution of a hydrogen atom of an amino group, imino group, carboxy group, hydroxy group, thiol group, benzene ring, imidazole ring, or indole ring in the side chain of a natural amino acid with -Rf or -Z³-(Rf)n4 can be performed according to a general synthesis reaction such as an ester reaction.

As the fluorine-containing peptide according to the present invention, a peptide containing at least one amino acid residue whose side chain is -Z¹-(Rf)n3 may be exemplified. Among amino acid residues constituting the peptide, at least one side chain may be -Z¹-(Rf)n3, and side chains of all amino acid residues may be -Z¹-(Rf)n3. When one peptide molecule has two or more amino acid residues whose side chains are -Z¹-(Rf)n3, the plurality of -Z¹-(Rf)n3's may be of the same type or different types. In addition, in the peptide, the amino acid residue whose side chain is -Z¹-(Rf)n3 may be located at the N-terminal, at the C-terminal, or at a position other than a terminal.

The fluorine-containing peptide according to the present invention may be a peptide composed of 2 or more amino acids, and is preferably a peptide composed of 3 or more amino acids. The fluorine-containing peptide according to the present invention is preferably a peptide composed of 2 to 40 amino acids and more preferably a peptide composed of 3 to 20 amino acids.

The amino acid sequence of the fluorine-containing peptide according to the present invention is not particularly limited. For example, peptides with a higher proportion of hydrophobic (non-polar) side chains than polar side chains have a better ability to permeate a cell membrane. Therefore, the fluorine-containing peptide according to the present invention can also have an amino acid sequence including a relatively large number of amino acid residues having hydrophobic side chains. On the other hand, when the fluorine-containing peptide according to the present invention is used in linkage with a hydrophobic substance, the fluorine-containing peptide has an amino acid sequence including a relatively large number of amino acid residues having polar side chains, and thus it is possible to balance the hydrophobicity and the hydrophilicity of all molecules, and it is possible to further improve the ability to permeate a cell membrane.

The N-terminal of the fluorine-containing peptide according to the present invention may be protected with a protecting group for an amino group. The N-terminal protecting group is not particularly limited as long as it is a protecting group for an amino group, and for example, a protecting group for an amino group used in peptide synthesis can be used. Examples of protecting groups for amino groups include carbamate-based protecting groups such as a tert-butoxycarbonyl (Boc) group, 9-fluorenylmethyloxycarbonyl (Fmoc) group, benzyloxycarbonyl (Cbz) group, allyloxycarbonyl (Alloc) group, and 2,2,2-trichloroethoxycarbonyl (Troc) group. A tert-butoxycarbonyl (Boc) group or a 9-fluorenylmethyloxycarbonyl (Fmoc) group is preferable because deprotection can be performed under mild conditions.

The C-terminal of the fluorine-containing peptide according to the present invention may be protected with a protecting group. The C-terminal protecting group is not particularly limited as long as it is a protecting group for a carboxy group, and for example, a protecting group for a carboxy group used in peptide synthesis can be used. The protecting group for a carboxy group is specifically a protecting group selected from among groups represented by the following General Formula (p-1), a 2-(9,10-dioxo)anthryl methyl group, a benzyloxymethyl group, and a phenacyl group. In General Formula (p-1), R³ is an optionally substituted C₆₋₁₄ aryl group, and R⁴ and R⁵ are each independently a hydrogen atom or an optionally substituted C₆₋₁₄ aryl group. In addition, the black circle indicates a bonding site.

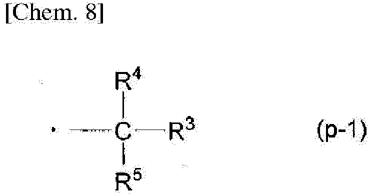

Examples of protecting groups for carboxy groups include a benzyl group, diphenylmethyl group, triphenylmethyl group, 4-nitrobenzyl group, 4-methoxybenzyl group, 2,4-dimethoxybenzyl group, 3,4-dimethoxybenzyl group, 4-methylbenzyl group, 2,6-dimethylbenzyl group, 3-chlorobenzyl group, 9-anthrylmethyl group, piperonyl group, 2-(9,10-dioxo)anthryl methyl group, benzyloxymethyl group, and phenacyl group. The protecting group for a C-terminal carboxy group is preferably a benzyl group or a triphenylmethyl group, and more preferably a benzyl group because deprotection can be performed under mild conditions.

In the fluorine-containing peptide according to the present invention, the amino acid residue whose side chain is not -Z¹-(Rf)n3 is not particularly limited, and it may be an amino acid residue of α-amino acids, an amino acid residue of β-amino acids, an amino acid residue of γ-amino acids, or an amino acid residue of δ-amino acids. In addition, it may be an amino acid residue of L-amino acids, or an amino acid residue of D-amino acids. The amino acid residue whose side chain is not -Z¹-(Rf)n3, which is contained in the fluorine-containing peptide according to the present invention, is preferably an amino acid constituting a protein, its D forms, or an amino acid residue of modified amino acids whose side chains are modified.

Examples of amino acids constituting a protein include glycine, alanine, valine, leucine, isoleucine, serine, threonine, phenylalanine, tyrosine, tryptophan, asparagine, glutamine, proline, aspartic acid, glutamic acid, lysine, arginine, and histidine. In addition, examples of modified amino acids in which amino acids constituting a protein are modified include amino acids in which a hydrogen atom of an amino group in the side chain of lysine, arginine, or histidine is substituted with a protecting group for an amino group or a Pbf(N-ω-(2,2,4,6,7-pentamethyldihydrobenzofuran-5-sulfonyl) group exemplified above; amino acids in which a hydrogen atom of a carboxy group in the side chain of aspartic acid or glutamic acid is substituted with a protecting group for a carboxy group or an alkyl group of a tert-butyl group exemplified above; and amino acids in which a hydrogen atom of a thiol group of cysteine is substituted with a benzyl group.

Examples of fluorine-containing peptides according to the present invention include a tripeptide represented by the following General Formula (11). In General Formula (11), Rf and n3 are the same as Rf and n3 in General Formula (1), and Z² and Rh are the same as Z² and Rh in General Formula (2).

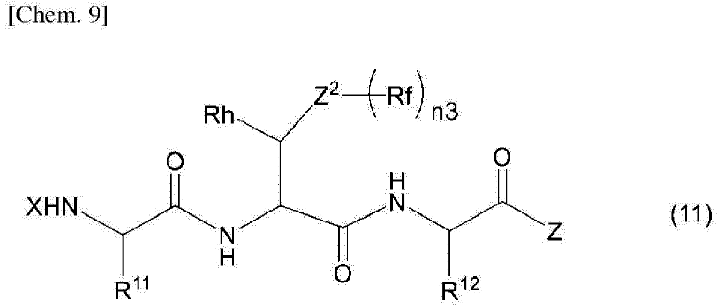

In General Formula (11), R¹¹ and R¹² are each independently a C₁₋₆ alkyl group or a benzyl group. In the tripeptide of General Formula (11), R¹¹ and R¹² are each independently preferably a methyl group or a benzyl group, and R¹¹ is particularly preferably a methyl group, and R¹² is particularly preferably a benzyl group.

In General Formula (11), X is a 9-fluorenylmethyloxycarbonyl group (Fmoc) or a tert-butoxycarbonyl group (Boc).

In General Formula (11), Zis a C₁₋₆ alkoxy group, hydroxyl group, or amino group. When Z is a C₁₋₆ alkoxy group, Z is particularly preferably a methoxy group.

The fluorine-containing peptide according to the present invention can be produced by a general peptide synthesis method, except that an amino acid having at least -Z¹-(Rf)n3 in the side chain is used as a raw material amino acid. For example, a peptide solid-phase synthesis method can be used. The fluorine-containing peptide can be easily synthesized using an amino acid whose side chain is -Z¹-(Rf)n3 as a raw material using an automatic peptide synthesizer. The fluorine-containing amino acid is preferably an amino acid whose side chain is -Z¹-(Rf)n3.

A peptide can be produced by sequentially condensing an amino acid with a protected amino group with an amino acid with the C-terminal bonded to a solid phase and then releasing the peptide from the solid phase. An amino acid raw material in which an amino group is protected with a Boc group or an Fmoc group is preferably used. An amino acid raw material whose side chain functional group is protected with a protecting group is preferably used. Examples of protecting groups for side chain functional groups include a Boc group, triphenylmethyl group, benzyl group, and 2,2,5,7,8-pentamethylchroman-6-sulfonyl (Pmc) group.

Examples of condensing agents that form peptide bonds include N,N-dicyclohexylcarbodiimide (DCC), 1-ethyl-3-(3 '-dimethylaminopropyl)carbodiimide (WSC), benzotriazol-1-yloxy-trisdimethylaminophosphonium hexafluorophosphate (BOP), benzotriazol-1-yloxytrispyrrolidinophosphonium hexafluorophosphate (pyBOP), 2-(1H-benzotriazol-1-yl)-1,1,3,3-tetramethyluronium hexafluorophosphate (HBTU), 2-(1H-benzotriazol-1-yl)-1,1,3,3-tetramethyluronium tetrafluoroborate, and 1-cyano-2-ethoxy-2-oxoethylideneaminooxy)dimethylaminomorpholinocarbenium hexafluorophosphate (COMU). In addition, N-hydroxybenzotriazole (HOBt), (hydroxyimino)ethyl cyanoacetate (oxyma) and the condensing agent can also be used by mixing them at a preferable ratio.

A method of activating a carboxy terminal may be used to form a peptide bond, and examples of activating agents include N-hydroxysuccinimide, p-nitrophenyl ester, and pentafluorophenyl ester. Examples of bases used when peptide bonds are formed include trimethylamine and diisopropylethylamine (DIPEA). Examples of solvents used in the peptide bond formation reaction include chloroform, dichloromethane (DCM), dichloroethane (DCE), acetonitrile (MeCN), N,N-dimethylformamide (DMF), and dimethyl sulfoxide (DMSO).

The Boc group and Fmoc group, which are amino terminal protecting groups for amino groups of peptides or amino acids, can be removed with trifluoroacetic acid (TFA) or piperidine. Protecting groups for side chain functional groups of amino acid residues of peptides can be removed using, for example, TFA, hydrogen fluoride (HF), trifluoromethane sulfonic acid or the like.

In addition, in the peptide solid-phase synthesis method, as a method of releasing peptides or peptides having protecting groups attached to side chain functional groups of amino acid residues from the peptide solid-phase synthetic resin, for example, TFA can be used. Release of peptides from the peptide solid-phase resin and release of protecting groups for side chain functional groups of amino acid residues can be performed simultaneously in the same reaction system, or performed independently. As the peptide solid-phase synthetic resin for peptide solid-phase synthesis, for example, commonly commercially available resins such as 4-hydroxymethyl-3-methoxyphenoxybutyric acid-benzhydrylamine-polystyrene resins, p-benzyloxybenzyl alcohol-polystyrene resins, and oxime resins can be used.

Target peptides or intermediates can be isolated and purified by various methods, for example, ion chromatography, gel filtration chromatography, reversed-phase chromatography, normal phase chromatography, recrystallization, extraction, and fractional crystallization. In addition, the peptides obtained in this manner can be converted into respective salts by general methods.

The protecting group for an amino group or a carboxy group of the produced fluorine-containing peptide can also be deprotected as necessary. Deprotection can be performed by general methods depending on the type of the protecting group.

Since the fluoroalkyl group and -SF5, -SF₄-CR¹⁰¹R¹⁰²-CR¹⁰³R¹⁰⁴Cl contain a large number of fluorine atoms, the fluorine-containing peptide according to the present invention is highly effective at permeating a cell membrane. In addition, since the fluorine-containing peptide has a significantly different structure from natural peptides, it is hardly degraded with peptidase. Using these properties, the fluorine-containing peptide according to the present invention is expected to be used as a physiologically active substance in the field of pharmaceuticals. For example, the fluorine-containing peptide according to the present invention can be expected to be used as a DDS carrier for delivery of medicinal components to target cells. When the fluorine-containing peptide according to the present invention is added to a functional component that exhibits some physiological activity when uptaken into target cells in living organisms in order to prevent its functions from being impaired, it is possible to improve efficiency of the functional component uptaken into the target cells. The functional component may be a peptide, a protein, or a low-molecular-weight compound. In addition, when some side chains of amino acid residues constituting the functional peptide that exhibits physiological activity are substituted with -Z¹-(Rf)n3 within a range that does not impair functions of the functional peptide, it is possible to improve the ability to permeate a cell membrane of the functional peptide and increase the time the functional peptide is retained in cells.

Even when a CPP-active peptide (medicinal moiety) is linked in order to impart the ability to permeate a cell membrane, synthesis using an automatic synthesis device is cost-effective and practical.

For non-natural amino acids as described in PCT International Publication No. WO 2021/002407, it is necessary to perform a stereoselective reaction or optical resolution, and synthesizing various fluorine-containing amino acid derivatives takes much time and effort. In addition, synthesis of peptides derived from such non-natural amino acids described in PCT International Publication No. WO 2021/002408 also takes much time and effort.

On the other hand, the peptide according to the present invention can be derived from natural amino acids, and amino acids with defined three-dimensional structures can be synthesized without optical resolution. In addition, when synthesis can be performed by separately preparing a fluorine-containing unit and reacting it with a natural amino acid, various fluorine-containing amino acids can be synthesized in the same production process, which is advantageous in terms of cost.

### [Examples]

Hereinafter, the present invention will be described with reference to examples, but the present invention is not limited to these examples.

The NMR device used for analysis of examples and comparative examples was JNM-ECZ400S (400 MHz, commercially available from JEOL Ltd.), and the reference value was 0 PPM for tetramethylsilane in ¹H NMR, and -162 PPM for C₆F₆ in ¹⁹F NMR.

In this specification, the following abbreviations are used.
Bn: benzyl
Boc: t-butoxycarbonyl
All: allyl
Phth: phthaloyl
EtzO: diethyl ether
Fmoc: 9-fluorenylmethyloxycarbonyl
THF: tetrahydrofuran
TMS: trimethylsilyl
C₄F₉: 1,1,2,2,3,3,4,4,4-nonafluorobutyl
C₆F₁₃: 1,1,2,2,3,3,4,4,5,5,6,6,6-tridecafluorohexyl
C₈F₁₇: 1,1,2,2,3,3,4,4,5,5,6,6,7,7,8,8,8-heptadecafluorooctyl
COMU: (1-cyano-2-ethoxy-2-oxoethylideneaminooxy)
dimethylaminomorpholinocarbenium hexafluorophosphate (CAS RN: 1075198-30-9)
oxyma: ethyl (hydroxyimino) cyanoacetate (CAS RN: 3849-21-6)
DIPEA: diisopropylethylamine

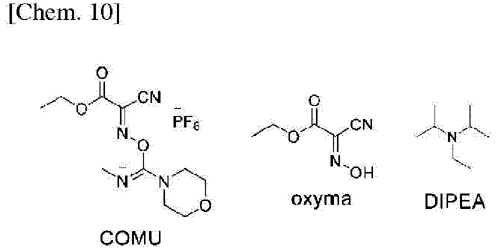

### [Production Example 1]

### Synthesis of RF(C8) group-containing amino acid (Fmoc-Asp(C₈F₁₇)-OH: 2b)

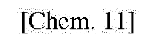

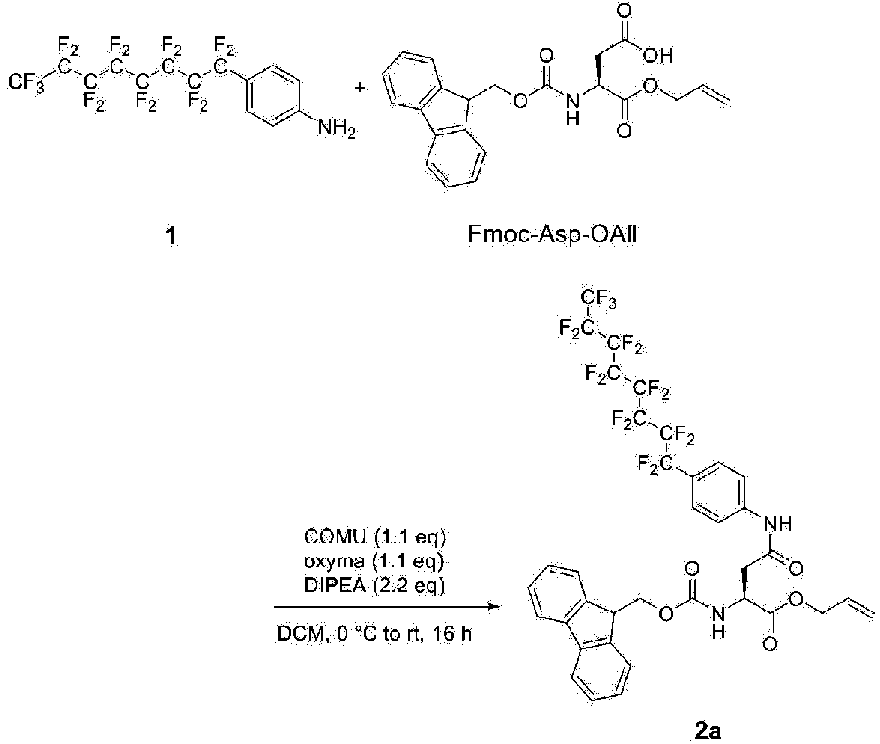

4-(Perfluorooctyl)aniline (Compound (1)) was synthesized according to the method described in Org. Lett., 2019, 21,6481.

In addition, in a dry 25 mL two-necked flask, 150 mg (0.3 mmol) of Compound (1) and 130 mg (0.33 mmol, 1.1 equivalents) of Fmoc-Asp-OAll were dissolved in 6 mL of dichloromethane, 141 mg (0.33 mmol, 1.1 equivalents) of COMU, 47 mg (0.33 mmol, 1.1 equivalents) of oxyma, and 85.3 mg (0.66 mmol, 2.2 equivalents) of DIPEA were added, and the mixture was stirred at room temperature for 18 hours. Then, the reaction mixture was quenched with HCl (1N) and extracted three times with dichloromethane. The combined organic phase was concentrated under a reduced pressure and then diluted with ethyl acetate, and washed with HCl (1N), a saturated sodium bicarbonate solution, and a saturated saline solution. The washed organic phase was dried with sodium sulfate, filtered, and then concentrated under a reduced pressure to obtain a crude product of Compound (2a). The crude product was dissolved in acetone and then reprecipitated and purified using hexane to obtain a white solid (173 mg, 0.19 mmol) of pure Compound (2a) (Fmoc-Asp(C₈F₁₇)-OAll) (yield 64.9%)

### Compound (2a):

¹H NMR(400 MHz, DMSO-D₆) δ=7.87-7.77(m, 5H), 7.65-7.57(m, 4H), 7.37-7.22(m, 4H), 5.82(m, 1H), 5.26(d, J=17.1 Hz, 1H), 5.12(d, J=17.1 Hz, 1H), 4.54(m, 3H), 4.29-4.18(m, 3H), 2.94-2.54(m, 2H).
¹⁹F NMR(376 MHz, DMSO-D₆) δ=-80.0(s, 3F), -108.8(s, 2F), -121.0(s, 2F), -121.6(s, 6F), -122.3(s, 2F), -125.7(s, 2F).

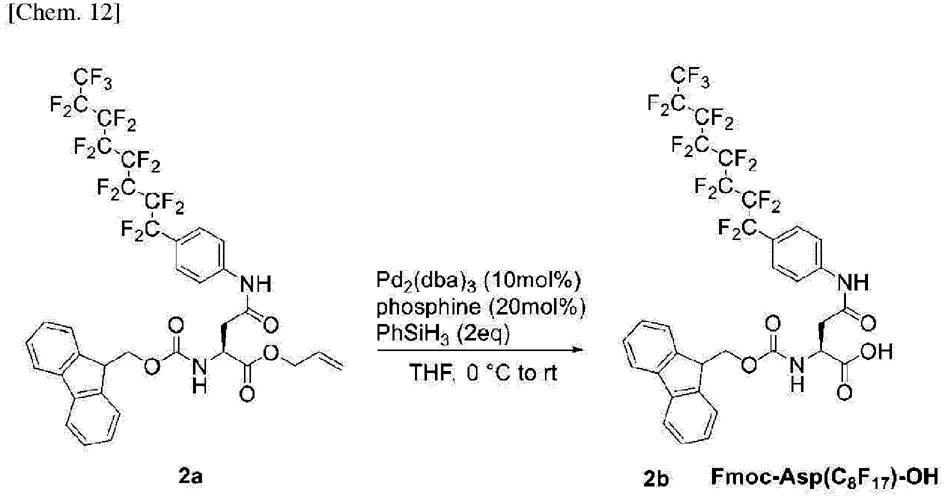

Tris(dibenzylideneacetone)dipalladium (0) (10 mol%) and phosphine (20 mol%) were added to a THF (2 mL) solution of Compound (2a) (173 mg, 0.2 mmol) at 0°C, the mixture was stirred, phenylsilane (2 equivalents) was additionally added, the temperature was then raised to room temperature, and the mixture was stirred for 2 hours. Then, the reaction mixture was quenched with HCl (1N) (10 mL) and extracted twice with dichloromethane. The organic phase was concentrated under a reduced pressure, the obtained crude product was purified through silica gel column chromatography (chloroform: methanol=8:1 (volume ratio)) to obtain a yellow solid (139 mg, 0.16 mmol, yield 82%) of Fmoc-Asp(C₈F₁₇)-OH (Compound (2b)).

### Compound (2b):

¹H NMR(500 MHz, DMSO-D₆) 8=10.37(s, 1H), 7.85-7.21(m, 12H), 4.44(brs, 1H), 4.52-4.22(m, 3H), 2.90-2.68(m, 2H).
¹⁹F NMR(376 MHz, DMSO-D₆) δ=-80.06(s, 3F), -108.83(s, 2F), -122.36 to -120.96(m, 10F), -125.41(s, 2F).

### [Production Example 2]

### Synthesis of RF(C8) group-containing amino acid (Fmoc-Asp(bis-C₈F₁₇)-OAll: 4)

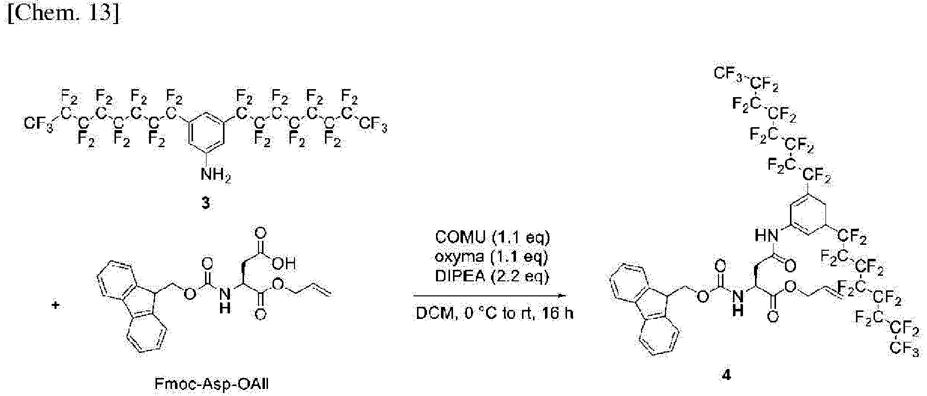

First, 3,5-bis(perfluorooctyl)aniline (Compound (3)) was synthesized according to the method described in Tetrahedron., 2002, vol. 58 (20), p.3977.

Next, a crude product of Fmoc-Asp(bis-C₈F₁₇)-OAll (Compound (4)) was obtained by the same procedure as in Production Example 1 using Compound (3) (930 mg, 0.1 mmol) in place of 4-(perfluorooctyl)aniline. The crude product was purified through silica gel chromatography (ethyl acetate/hexane=1/4 (volume ratio)) to obtain pure Compound (4) (940 mg, 0.07 mmol, yield 72%)

### Compound (4):

¹H NMR(400 MHz, CDCl₃) δ=7.76(d, J=7.3 Hz, 2H), 7.59(m, 3H), 7.35(dt, J=34.8, 7.5 Hz, 6H), 5.94-5.82(m, 3H), 5.35-5.23(m, 3H), 4.43-4.21(m, 3H), 2.98(dd, J=74.3, 17.2 Hz, 2H).
¹⁹F NMR(376 MHz, CDCl₃) δ=-80.44(s, 6F), -110.83(s, 4F), -122.52 to -121.03(m, 20F), -125.97(s,4F).

### [Production Example 3]

### Synthesis of RF(C4) group-containing amino acid (Fmoc-Asp(bis-C₄F₉)-OH: 6b)

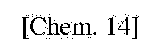

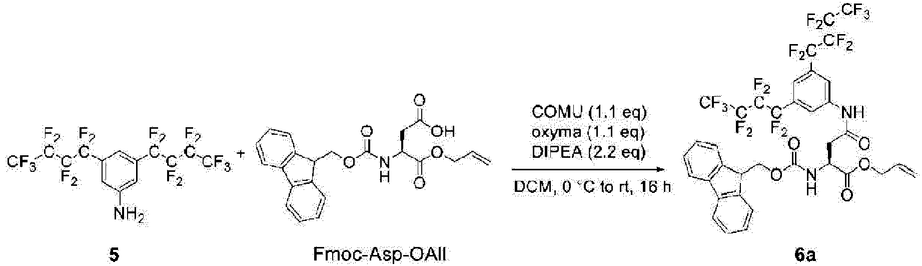

First, 3,5-bis(perfluorobutyl)aniline (Compound (5)) was synthesized using C₄F₉I in place of C₈F₁₇I according to the method described in Tetrahedron., 2002, vol. 58 (20), p. 3977.

Next, a crude product of Fmoc-Asp(bis-C₄F₉)-OAll (Compound (6a)) was obtained by the same procedure as in Production Example 1 using Compound (5) (1.1 g, 2 mmol) in place of 4-(perfluorooctyl)aniline. The crude product was purified through silica gel chromatography (ethyl acetate/hexane=1/10 (volume ratio)) to obtain pure Compound (6a) (370 mg, 0.39 mmol, yield 19.5%).

### Compound (6a):

¹H NMR(400 MHz, ACETONE-D₆) δ=9.92(s, 1H), 7.82-7.61(m, 6H), 7.30(dt, J=42.2, 7.4 Hz, 4H), 6.90(d, J=8.2 Hz, 1H), 5.88(dq, J=22.8, 5.3 Hz, 1H), 5.32-5.11(m, 2H), 4.75(dd, J=14.4, 6.2 Hz, 1H), 4.62(d, J=6.9 Hz, 2H), 4.37-4.21(m, 3H), 3.11(d, J=6.4 Hz, 2H).
¹⁹F NMR(376 MHz, ACETONE-D₆) δ=-81.81(s, 6F), -111.45(s, 4), -123.15(s, 4F), - 126.09(s,4F).

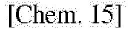

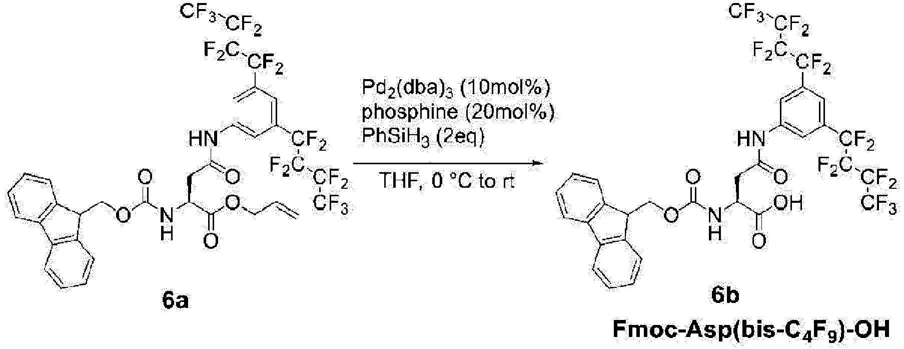

Subsequently, Compound (6a) (180 mg, 0.2 mmol) was deallylated by the same method as in Production Example 1 to obtain Fmoc-Asp(bis-C₄F₉)-OH (Compound (6b)) (91 g, 0.11 mmol, yield 53%).

### Compound (6b):

¹H NMR(400 MHz, ACETONE-D₆) δ=9.81(1H), 7.89-6.91(m, 12H), 4.71(s, 1H), 4.31-4.25(m, 3H), 3.11-2.88(m, 2H).
¹⁹F NMR(376 MHz, ACETONE-D₆) δ=-81.75(s, 6F), -111.45(s, 4F), -123.12(s, 4F), - 126.07(s,4F).

### [Production Example 4]

### Synthesis of SF₅ group-containing amino acid (Fmoc-Asp(SF₅)-OH: 8b)

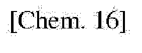

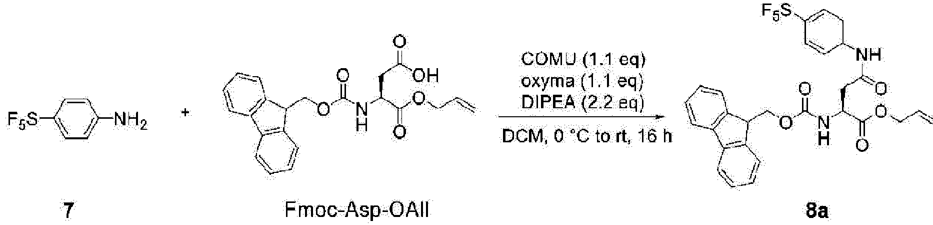

Fmoc-Asp(SF₅)-OAll (Compound (8a)) (882 mg, 1.48 mmol, yield 74%) was obtained by the same procedure as in Production Example 1 using 4-pentafluorosulfanylaniline (Compound (7)) (438 mg, 2 mmol) in place of 4-(perfluorooctyl)aniline. The product was subjected to the following deprotection without purification.

### Compound (8a):

¹H NMR(400 MHz, ACETONE-D₆) 8=9.75(s, 1H), 7.84-7.23(m, 12H), 5.94-5.84(m, 1H), 5.33-5.12(m, 2H), 4.84-4.72(m, 3H), 4.35-4.31(m, 3H), 3.41-3.22(m, 2H).
¹⁹F NMR(376 MHz, ACETONE-D₆) δ=88.14-86.65(m, 5F).

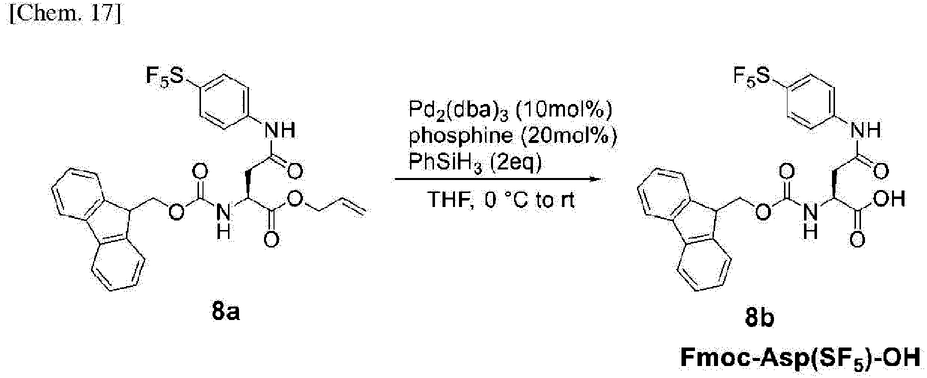

Subsequently, Compound (8a) (120 mg, 0.2 mmol) was deallylated by the same method as in Production Example 1 to obtain Fmoc-Asp(SF₅)-OH (Compound (8b)) (0.10 mmol, 55 mg, yield 49%).

### Compound (8b)

¹H NMR(400 MHz, ACETONE-D₆) δ=9.74(s, 1H), 7.78-6.90(m, 12H), 4.73(s, 1H), 4.35-4.20(m, 3H), 2.95-2.81 (m, 4H).
¹⁹F NMR(376 MHz, ACETONE-D₆) δ=86.7-85.1(m, 5F).

### [Production Example 5]

### Synthesis of branched chain RF(C₄) group-containing amino acid (Fmoc-Asp(tris-t-C₄F₉)-OH: 10b)

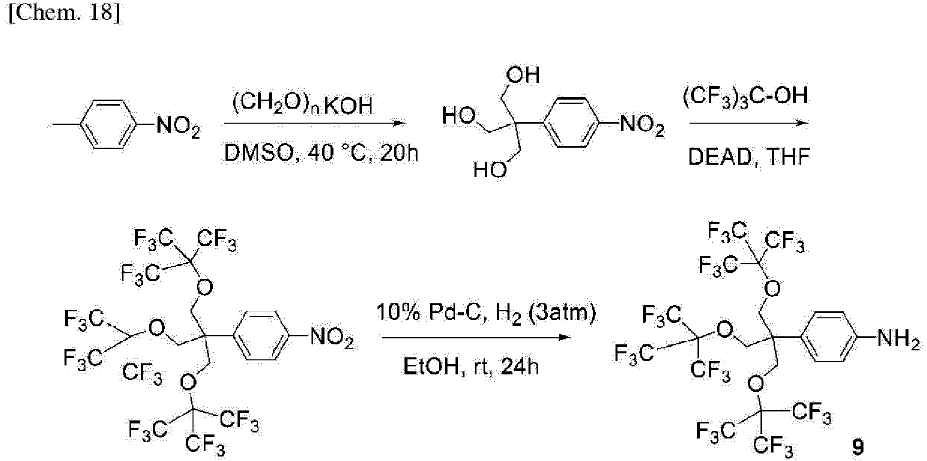

First, Compound (9) was synthesized according to the method described in Dalton Trans., 2012, vol. 41, p. 8368 and Electronic Supplementary Information, New J. Chem., 2017, vol. 41, p. 7729 according to the above reaction formula.

Next, a crude product of Fmoc-Asp(tris-t-C₄F₉)-OAll (Compound (10a)) was obtained by the same procedure as in Production Example 1 using a compound having a plurality of branched Rf groups (Compound (9)) (400 mg, 0.47 mmol) in place of 4-(perfluorooctyl)aniline. The crude product was purified through silica gel chromatography (ethyl acetate/hexane=1/10 (volume ratio)) to obtain pure Compound (10a) (300 mg, 0.24 mmol, yield 52%).

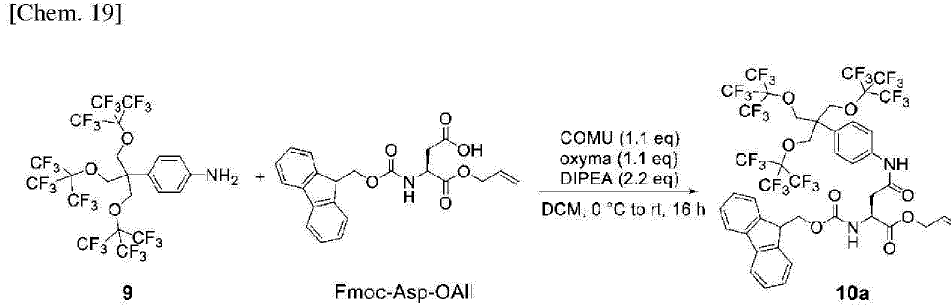

### Compound (10a)

¹H NMR(500 MHz, CDCl₃) δ=7.75(d, J=6.9 Hz, 2H), 7.60-7.28(m, 8H), 7.21(d, J=8.6 Hz, 2H), 6.09(d, J=8.0 Hz, 1H), 5.92-5.86(m, 1H), 5.34-5.21(m, 2H), 4.70(d, J=5.2 Hz, 3H), 4.44(dd, J=10.3, 7.4 Hz, 2H), 4.36(s, 6H), 4.23(t, J=7.2 Hz, 1H), 3.07(dd, J=16.5, 12.0 Hz, 2H).
¹⁹F NMR(471 MHz, CDCl₃) δ=-70.12(s, 27F).

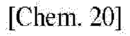

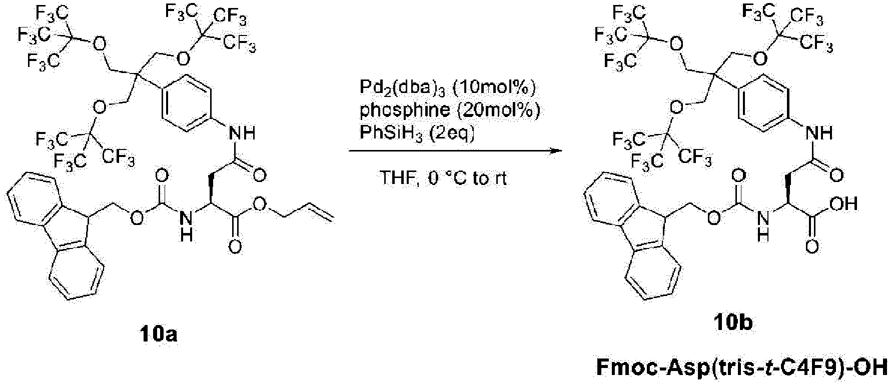

Subsequently, Compound (10a) (248 mg, 0.2 mmol) was deallylated by the same method as in Production Example 1 to obtain Fmoc-Asp(tris-t-C₄F₉)-OH (Compound (10b)) (108 mg, 0.091 mmol, yield 45%).

### Compound (10b):

¹H NMR(400 MHz, ACETONE-D₆) δ=7.74-7.60(m, 8H), 7.36-7.24(m, 4H)4.61(s, 6H), 4.34-4.16(m, 4H), 2.92-2.81(m, 2H).
¹⁹F NMR(376 MHz, ACETONE-D₆) δ=-70.82(s, 27F).

### [Production Example 6]

### Synthesis of Dodecyl group-containing amino acid (Fmoc-Asp(C₁₂H₂₅)-OH: 12b)

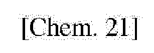

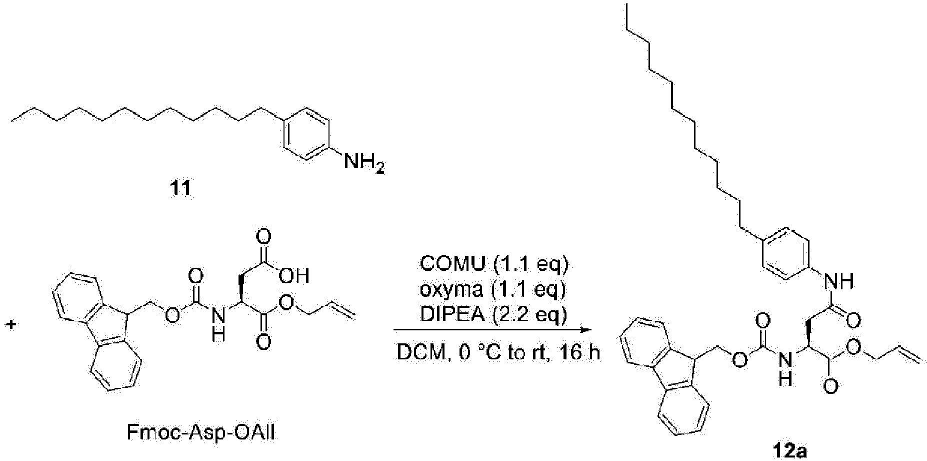

A crude product of Fmoc-Asp(C₁₂H₂₅)-OAll (Compound (12a)) was obtained by the same procedure as in Production Example 1 using 4-dodecylaniline (Compound (11)) (78 mg, 0.3 mmol) in place of 4-(perfluorooctyl)aniline. The crude product was purified through silica gel chromatography (ethyl acetate/hexane=1/10 (volume ratio)) to obtain pure Compound (12a) (172 mg, 0.27 mmol, yield 90%).

### Compound (12a):

¹H NMR(500 MHz, CDCl₃) δ=7.74(d, J=7.4 Hz, 2H), 7.59-7.57(m, 2H), 7.38-7.26(m, 6H), 7.11(d, J=8.6 Hz, 2H), 5.93-5.85(m, 1H), 5.34-5.21(m, 2H), 4.80-4.72(m, 2H), 4.69(s, 1H), 4.22(t, J=7.2 Hz, 3H), 3.04(dd, J=116.7, 15.9 Hz, 2H), 1.28-1.24(m, 25H).

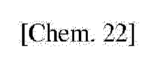

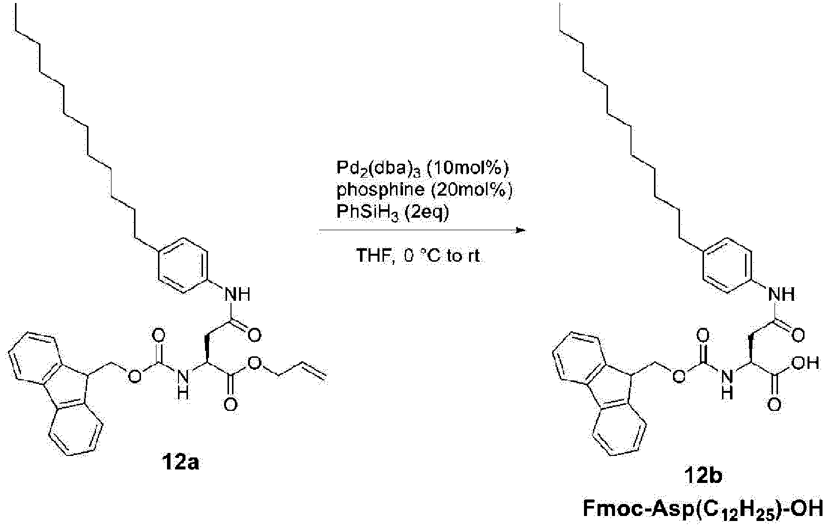

Subsequently, Fmoc-Asp(C₁₂H₂₅)-OAll Compound (12a) (135 mg, 0.211 mmol) was deallylated by the same method as in Example 1, and the obtained crude product was purified through silica gel column chromatography (ethyl acetate:hexane=1:10 (volume ratio)) to obtain Fmoc-Asp(C₁₂H₂₅)-OH (Compound (12b)) (71 mg, 0.119 mmol, yield 56%).

### Compound (12b)

¹H NMR(500 MHz, CDCl₃) δ=7.74-7.49(m, 6H), 7.13-6.94(m, 6H), 4.55(s, 1H), 4.37-4.20(m, 3H), 3.09-2.89(m, 2H), 1.28-1.21(m, 25H).

### [Production Example 7]

### Synthesis of RF group-containing amino acid (Fmoc-Asp(C₄F₉)-OH: 14b, Fmoc-Asp(C₆F₁₃)-OH: 16b, Fmoc-Asp(CH₂CH₂C₆F₁₃)-OH: 18b, Fmoc-Asp(SF₄CF₂CF₂Cl)-OH: 20b)

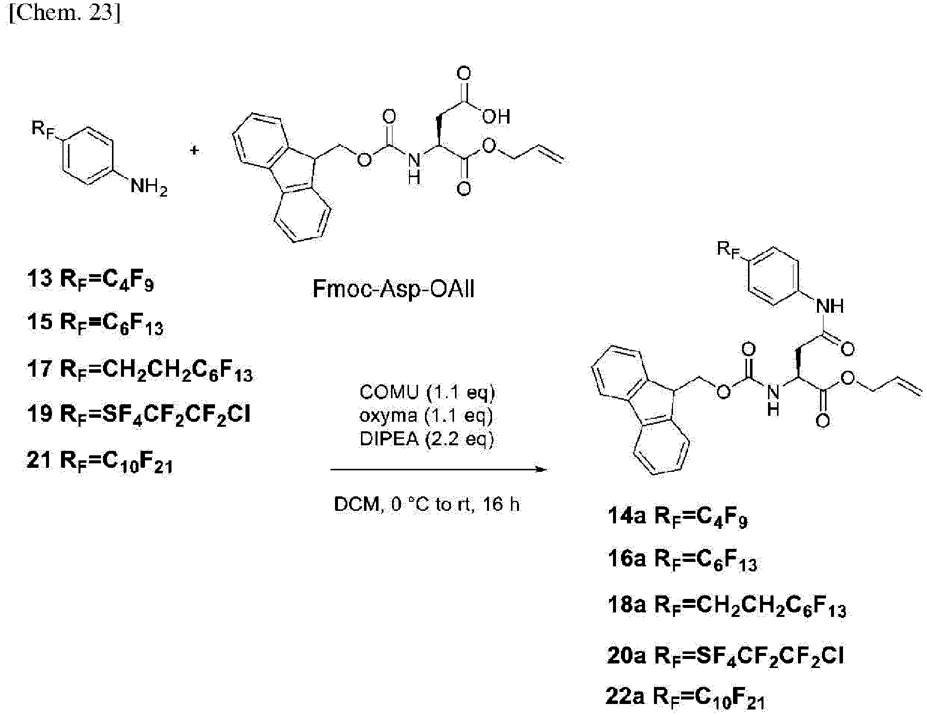

A crude product of Fmoc-Asp(C₄F₉)-OAll (Compound (14a)) was obtained by the same procedure as in Production Example 1 using 4-(perfluorobutyl)aniline (Compound (13)) (731 mg, 4.1 mmol) in place of 4-(perfluorooctyl)aniline. The crude product was purified through silica gel chromatography (ethyl acetate/hexane=1/10 (volume ratio)) to obtain pure Compound (14a) (1, 072 mg, 2.5 mmol, yield 61%).

### Compound (14a):

¹H NMR(400 MHz, CDCl₃)δ=7.75 (d, J=7.4 Hz, 2H), 7.62(d, J=7.4 Hz, 2H), 7.60-7.50(m, 4H), 7.45-7.30(m, 4H), 5.97-5.73(m, 1H), 5.28(m, 2H), 4.77-4.57(m, 3H), 4.52-4.29(m, 2H), 4.22(t, J=6.5 Hz, 1H), 3.17-2.95(m, 2H).
¹⁹FNMR(376MHz, CDCl₃) δ=-80.86(s, 3F), -110.46(s, 2F), -122.65(s, 2F), -125.49(s, 2F).

In addition, a crude product of Fmoc-Asp(C₆F₁₃)-OAll (Compound (16a)) was obtained by the same procedure as in Production Example 1 using 4-(perfluorohexyl)aniline (Compound (15)) (411 mg, 1.0 mmol) in place of 4-(perfluorooctyl)aniline. The crude product was purified through silica gel chromatography (ethyl acetate/hexane=1/10 (volume ratio)) to obtain pure Compound (16a) (575 mg, 0.73 mmol, yield 73%).

### Compound (16a):

¹H NMR(400 MHz, CDCl₃) δ=7.77(d, J=8.3 Hz, 2H), 7.59(d, J=7.1 Hz, 2H), 7.36(dt, J=34.3, 8.1 Hz, 8H), 5.90(m, J=10.6, 4.9 Hz, 1H), 5.79(d, J=7.2 Hz, 1H), 5.31(m, 2H), 4.74(m, 3H), 4.41(s, 2H), 4.23(t, J=7.0 Hz, 1H), 3.43-3.20(m, 2H).
¹⁹F NMR(376 MHz, CDCl₃) δ=-80.6(s, 3F), -110.3(s, 2F), -125.9 to -121.7(m, 8F).

In addition, a crude product of Fmoc-Asp(CH₂CH₂C₆F₁₃)-OAll (Compound (18a)) was obtained by the same procedure as in Production Example 1 using 4-(3,3,4,4,5,5,6,6,7,7,8,8,8-tridecafluorooctyl)aniline (Compound (17)) (500 mg, 1.14 mmol) in place of 4-(perfluorooctyl)aniline. The crude product was purified by recrystallization (acetone/cyclohexane=1/10) to obtain pure Compound (18a) (745 mg, 0.91 mmol, yield 80%).

### Compound (18a):

¹H NMR(500 MHz, ACETONE-D₆) δ=9.31(s, 1H), 7.85(d, J=7.4 Hz, 2H), 7.69(d, J=7.4 Hz, 2H), 7.61(d, J=8.0 Hz, 2H), 7.39(t, J=7.4 Hz, 2H), 7.31-7.27(m, 4H), 6.91(d, J=8.6 Hz, 1H), 5.92(qd, J=11.0, 5.3 Hz, 1H), 5.36-5.32(m, 1H), 5.17(dd, J=10.9, 1.1 Hz, 1H), 4.74-4.70(m, 1H), 4.64-4.63(m, 2H), 4.37-4.23(m, 3H), 3.05-2.90(m, 3H), 2.58-2.47(m, 2H).
¹⁹F NMR(471 MHz, ACETONE-D₆) δ=-80.70(t, J=10.1 Hz, 3F), -113.58 to -114.27(m, 2F), -121.49(s, 2F), -122.46(s, 2F), -123.02(s, 2F), -125.80(td, J=15.0, 7.1 Hz, 2F).

Here, 4-(3,3,4,4,5,5,6,6,7,7,8,8,8-tridecafluorooctyl)aniline (Compound (17)) was synthesized using 4-nitrobenzenediazonium tetrafluoroborate as a raw material by hydrogenation after induction into a coupled product by the Heck reaction according to the method described in Eur. J. Org. Chem. 2001, p. 1121-1128.

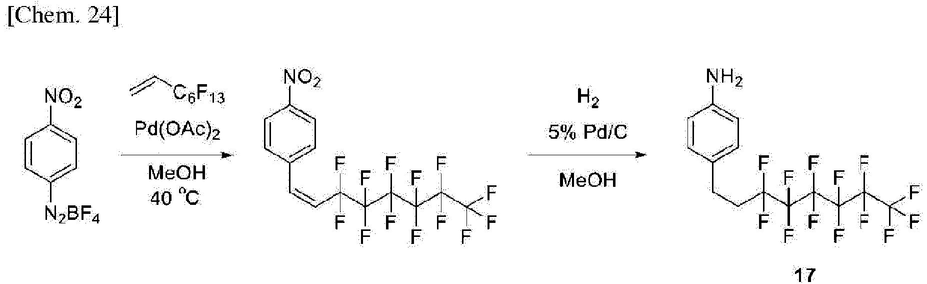

### Compound (17):

¹H NMR(500 MHz, ACETONE-D₆) 6=7.06-6.93(m, 2H), 6.69-6.56(m, 2H), 2.82-2.74(m, 2H), 2.53-2.32(m, 2H).
¹⁹F NMR(471 MHz, ACETONE-D₆) δ-81.61(s, 3F), -114.91(s, 2F), -122.40(s, 2F), - 123.37(s, 2F), -124.00(s, 2F), -126.71(s, 2F).

In addition, a crude product of Fmoc-Asp(SF₄CF₂CF₂Cl)-OAll (Compound (20a)) was obtained by the same procedure as in Production Example 1 using 4-[(3-chloro-1,1,2,2,3,3-hexafluoro-propyl)-tetrafluoro-1,6-sulfanyl]aniline (Compound (19)) (350 mg, 0.89 mmol) in place of 4-(perfluorooctyl)aniline. The crude product was purified through silica gel chromatography (ethyl acetate/hexane=1/10 (volume ratio)) to obtain pure Compound (20a) (321 mg, 0.45 mmol, yield 51%).

### Compound (20a):

¹H NMR(400 MHz, CDCl₃) δ=7.77(d, J=8.3 Hz, 2H), 7.59(d, J=7.1 Hz, 2H), 7.36(dt, J=34.3, 8.1 Hz, 8H), 5.90(m, J=10.6, 4.9 Hz, 1H), 5.79(d, J=7.2 Hz, 1H), 5.31(m, 2H), 4.74(m, 3H), 4.41(s, 2H), 4.23(t, J=7.0 Hz, 1H), 3.43-3.20(m, 2H).
¹⁹F NMR(376 MHz, CDCl₃) 8=-80.6(s, 3F), -110.3(s, 2F), -125.9 to -121.7(m, 8F).

Here, 4-[(3-chloro-1,1,2,2,3,3-hexafluoro-propyl)-tetrafluoro-1,6-sulfanyl]aniline (Compound (19)) was synthesized as follows.

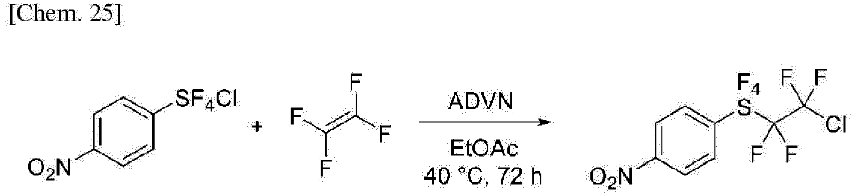

Under a nitrogen atmosphere, a solution in which 4-(chlorotetrafluoro-λ⁶-sulfanyl)nitrobenzene (0.20 mmol) and 2,2'-azobis-2-methylbutyronitrile and 2,2'-azobis-2,4-dimethylvaleronitrile (ADVN) (10 mol%) were dissolved in DCE (0.1 M) was stirred in a microwave vial (10 mL) and then cooled to 0°C. Next, tetrafluoroethylene (TFE) was filled into the solution (1.0 atm) by bubbling. After stirring in an oil bath at 40°C for 72 hours, insoluble solids were removed by filtration, and the filtrate was evaporated under a reduced pressure. The obtained crude product was purified through flash silica gel column chromatography (n-hexane/EtOAc=9/1 (volume ratio)) to obtain 4-(2-chlorotetrafluoroethyltetrafluoro-λ⁶-sulfanyl)nitrobenzene (0.040 g, yield 55%) as colorless crystals.

¹H NMR(400 MHz, CDCl₃) δ=8.33(d, J=8.7 Hz, 2H), 8.00(d, J=9.2 Hz, 2H).
¹³C NMR(100 MHz, CDCl₃) δ=159.4(quint, J_{C-F}=21.9 Hz), 149.2, 127.7(quint, J_{C-F}=4.8 Hz), 124.2, 122.1(tt, J_{C-F}=303.4, 35.6 Hz), 121.3(ttquint, J_{C-F}=312.1, 37.6, 36.6 Hz).
¹⁹F NMR(376 MHz, CDCl₃) δ=48.(m, 4F), -68.1(t, J=10.7 Hz, 2F), -90.7(m, 2F).

Iron (11 equivalents) and a saturated ammonium chloride aqueous solution (5 mL) were added to a solution in which 4-(2-chlorotetrafluoroethyltetrafluoro-λ⁶-sulfanyl)nitrobenzene (0.98 mmol) was dissolved in ethanol (20 mL), and the mixture was stirred in an oil bath at 80°C for 2 hours. Then, insoluble solids were removed by Celite filtration, and the filtrate was evaporated under a reduced pressure. The obtained crude product was dissolved in ethyl acetate and washed with water, the aqueous layer was extracted twice with ethyl acetate, the combined organic extract was then dried with sodium sulfate, and the solvent was evaporated under a reduced pressure. The obtained crude product was purified through flash silica gel column chromatography (n-hexane/EtOAc=4/1 (volume ratio)) to obtain desired Compound (19) (0.311 g, yield 95%) as a light yellow liquid.

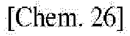

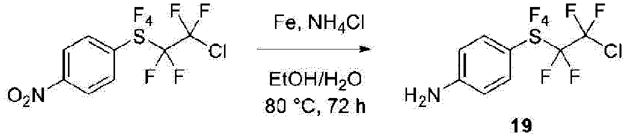

¹H NMR(400 MHz, CDCl₃) δ=7.55(d, J=8.7 Hz, 2H), 6.58(d, J=9.1 Hz, 2H), 3.97(br, 2H) ¹⁹F NMR(376 MHz, CDCl₃) δ=49.5(m, 4F), -67.9(t, J=10.8 Hz, 2F), -90. 7(m, 2F).

In addition, a crude product of Fmoc-Asp(C₁₀F₂₁)-OAll (Compound (21)) was obtained by the same procedure as in Production Example 1 using 4-(perfluorodecyl)aniline (Compound (15)) (611 mg, 1.0 mmol) in place of 4-(perfluorooctyl)aniline. The crude product was purified through silica gel chromatography (ethyl acetate/hexane=1/10 (volume ratio)) to obtain pure Compound (21) (810 mg, 0.82 mmol, yield 82%).

### Compound (21):

¹H NMR(400 MHz, ACETONE-D6) δ7.90-7.23(m, 12H), 5.93-5.84(m, 1H), 5.3 1 (dd, J=17.2, 1.6 Hz, 1H), 5.13(d, J=10.5 Hz, 1H), 4.73(t, J=6.0 Hz, 1H), 4.61(d, J=5.0 Hz, 2H), 4.36-4.19(m, 3H), 3.11-3.05(m, 2H).
¹⁹F NMR(376 MHz, ACETONE-D6) δ-81.6(s, 3F), -110.2(t, J=14.4 Hz, 2F), -121.6-123.1(m, 14F), -126.6(s, 2F).

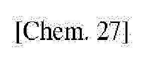

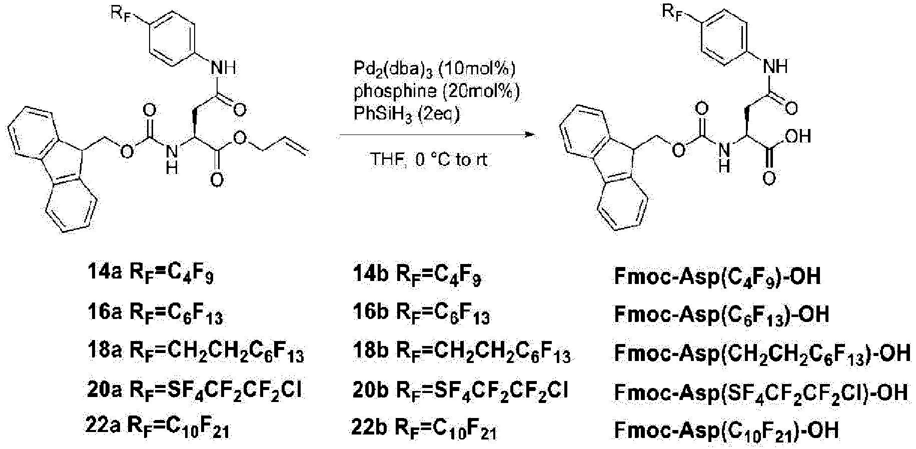

Subsequently, Compound (14a) (1,000 mg, 1.45 mmol) was deallylated by the same method as in Production Example 1 to obtain Fmoc-Asp(C₄F₉)-OH (Compound (14b)) (432 mg, 0.67 mmol, yield 46%).

### Compound (14b):

¹H NMR(400 MHz, ACETONE-D₆) δ=9.8(s, 1H), 8.0-7.8(m, 2H), 7.6(m, 2H), 7.3(dt, J=42.4, 7.1 Hz, 8H), 6.8(d, J=8.2 Hz, 1H), 4.7-4.6(m, 1H), 4.5-4.0(m, 3H), 3.0-2.7(m, 2H).
¹⁹F NMR(376 MHz, ACETONE-D₆) δ=-81.8(s, 3F), -110.4(d, J=9.5 Hz, 2F), -123.2(s, 2F), -126.1(s, 2F).

In addition, Compound (16a) (1.8 g, 2.28 mmol) was deallylated by the same method as in Production Example 1 to obtain Fmoc-Asp(C₆F₁₃)-OH (Compound (16b)) (965 mg, 1.28 mmol, yield 56%).

### Compound (16b):

¹H NMR(400 MHz, ACETONE-D₆) δ=10.0(s, 1H), 8.0-7.8(m, 4H), 7.3(d, J=41.7, 8H), 6.9(m, 1H), 4.6(d, J=7.0 Hz, 1H), 4.29-4.20(m, J=27.4, 6.7 Hz, 3H), 3.0-2.7(m, 2H).
¹⁹F NMR(376 MHz, ACETONE-D₆) δ=-81.6(s, 3F), -110.2(d, 2F), -121.9(s, 2F), - 122.4(s, 2F), -123.3(s, 2F), -126.7(s, 2F).

In addition, Compound (18a) (654 mg, 0.80 mmol) was deallylated by the same method as in Production Example 1 to obtain Fmoc-Asp(CH₂CH₂C₆F₁₃)-OH (Compound (18b)) (597 mg, 0.77 mmol, yield 96%).

### Compound (18b):

¹H NMR(400 MHz, ACETONE-D₆) δ=10.0(s, 1H), 8.0-7.8(m, 4H), 7.3(d, J=41.7, 8H), 6.9(m, 1H), 4.6(d, J=7.0 Hz, 1H), 4.29-4.20(m, J=27.4, 6.7 Hz, 3H).
¹⁹F NMR(376 MHz, ACETONE-D₆) δ=-81.6(s, 3F), -110.2(d, 2F), -121.9(s, 2F), - 122.4(s, 2F), -123.3(s, 2F), -126.7(s, 2F).

In addition, Compound (20a) (310 mg, 0.16 mmol) was deallylated by the same method as in Production Example 1 to obtain Fmoc-Asp(SF₄CF₂CF₂Cl)-OH (Compound (20b)) (74.4 mg, 0.16 mmol, yield 32%).

### Compound (20b):

¹H NMR(400 MHz, ACETONE-D₆) δ=10.0(s, 1H), 8.0-7.8(m, 4H), 7.3(d, J=41.7, 8H), 6.9(m, 1H), 4.6(d, J=7.0 Hz, 1H), 4.29-4.20(m, J=27.4, 6.7 Hz, 3H).
¹⁹F NMR(376 MHz, ACETONE-D₆) δ=-81.6(s, 3F), -110.2(d, 2F), -121.9(s, 2F), - 122.4(s, 2F), -123.3(s, 2F), -126.7(s, 2F).

### [Production Example 8]

### Synthesis of RF group-containing amino acid (Fmoc-Asp(CH₂CH₂₋C₈F₁₇)-OH: 24b)

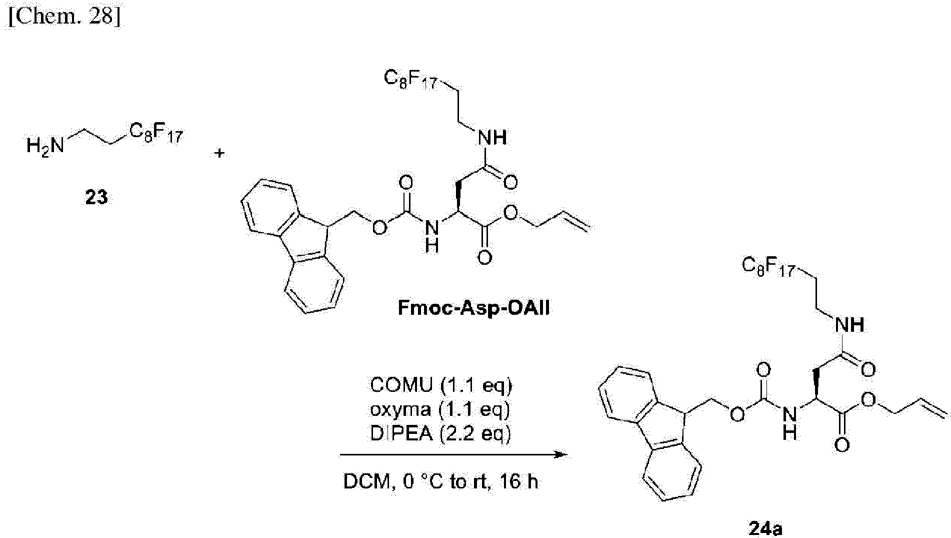

A crude product of Fmoc-Asp(CH₂CH₂C₈F₁₇)-OAll (Compound (24a)) was obtained by the same procedure as in Production Example 1 using 3,3,4,4,5,5,6,6,7,7,8,8,9,9,10,1β,10-heptadecafluoro-1-decanamine (Compound (23)) (3.79 mg, 8.08 mmol) in place of 4-(perfluorooctyl)aniline. The crude product was purified through silica gel chromatography (ethyl acetate/hexane=1/10 (volume ratio)) to obtain pure Compound (24a) (4.89 mg, 5.81 mmol, yield 72%).

### Compound (24a):

¹H NMR(400 MHz, CDCl₃) δ=7.75(d, J=7.4 Hz, 2H), 7.62(d, J=7.4 Hz, 2H), 7.60-7.50(m, 4H), 7.45-7.30(m, 4H), 5.97-5.73(m, 1H), 5.28(m, 2H), 4.77-4.57(m, 3H), 4.52-4.29(m, 2H), 4.22(t, J=6.5 Hz, 1H), 3.17-2.95(m, 2H).
¹⁹FNMR(376MHz, CDCl₃) δ=-80.86(s, 3F), -110.46(s, 2F), -122.65(s, 2F), -125.49(s, 2F).

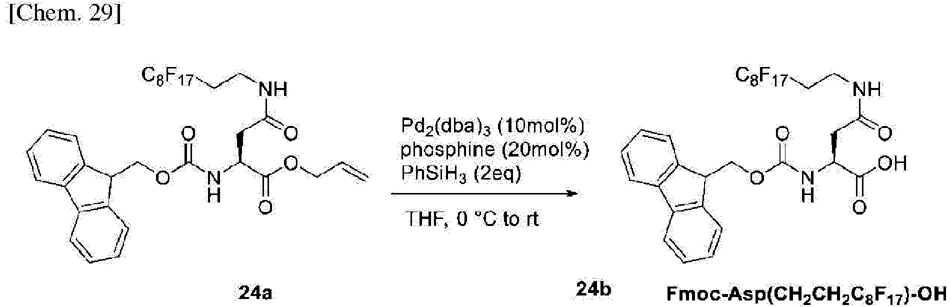

Subsequently, Compound (24a) (2.61 mg, 3.11 mmol) was deallylated by the same method as in Production Example 1 to obtain Fmoc-Asp(CH₂CH₂C₈F₁₇)-OH (Compound (24b)) (1.33 g, 1.67 mmol, yield 54%).

### Compound (24b):

¹H NMR(400 MHz, ACETONE-D6) δ=7.83(d, J=7.8 Hz, 2H), 7.68(d, J=7.3 Hz, 2H), 7.38(t, J=7.3 Hz, 2H), 7.31-7.27(m, 2H), 6.73(d, J=8.2 Hz, 1H), 4.56(dd, J=14.4, 5.7 Hz, 1H), 434-4.20(m, 3H), 3.52(t, J=6.4 Hz, 2H), 2.48(td, J=19.4, 7.2 Hz, 2H).
¹⁹F NMR(376 MHz, ACETONE-D6) δ=-81.5(t, J=10.1 Hz, 3F), -114.5(t, J=17.3 Hz, 2F), -122.1 to -124.0(m, 10F), -126.6(s, 2F).

### [Example 1]

### Synthesis of tripeptide H-Ala-Asp(C₈F₁₇)-Phe-NH₂

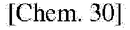

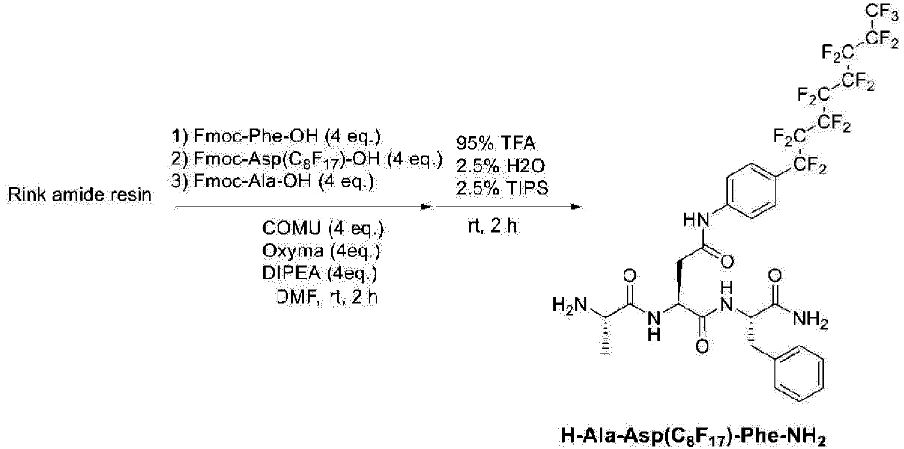

Rink-amide Resin (39 mg, 0.025 µmol) and DMF (2 mL) were put into a solid-phase synthesis tube and the resin was swollen by stirring at room temperature for 30 minutes. Next, a 20% piperidine/DMF solution (2 mL) was put into the tube and stirred for 3 minutes, and washing with DMF (2 mL) was then performed three times. A 20% piperidine/DMF solution (2 mL) was added again, the mixture was stirred for 12 minutes, and thus the Fmoc group was deprotected. Subsequently, Fmoc-Phe-OH (39 mg, 0.10 mmol, 4.0 equivalents), COMU (43 mg, 0.10 mmol, 4.0 equivalents), oxyma (14 mg, 0.10 mmol, 4.0 equivalents), and DIPEA (26 mg, 0.20 mmol, 8.0 equivalents) were added, the mixture was stirred at room temperature for 2 hours to perform an amino acid condensation reaction. Next, the reaction product was washed with DMF (2 mL) three times and Fmoc was then deprotected by the same method as above. The same operation was performed on Fmoc-Asp(C₈F₁₇)-OH (84 mg, 0.10 mmol, 4.0 equivalents) synthesized in Production Example 1 and Fmoc-Ala-OH (31 mg, 0.10 mmol, 4.0 equivalents). As a subsequent post-treatment, a cutting cocktail (TFA: triisopropylsilane:water=95:2.5:2.5 (volume ratio)) (2 mL) was added, stirring was performed at room temperature for 2 hours, and cutting out from the resin was performed to obtain a crude product containing H-Ala-Asp(C₈F₁₇)-Phe-NH₂. The crude product was vacuum-dried and then purified through reversed-phase chromatography (acetonitrile/water/TFA=75:25:0.1 to 99:1:0.1 (volume ratio)) to obtain H-Ala-Asp(C₈F₁₇)-Phe-NH₂ (5 mg, 5 µmol, yield 20%).

H-Ala-Asp(C₈F₁₇)-Phe-NH₂:
¹H NMR(500 MHz, DMSO-D₆) δ=7.43-7.36(m, 5H), 7.20-7.13(m, 4H), 5.27(t, J=7.2 Hz, 1H), 4.46(t, J=7.4 Hz, 1H), 4.32(t, J=7.2 Hz, 1H), 3.03-2.90(m, 2H), 2.81-2.59(m, 2H), 1.08(s, 3H).
¹⁹F NMR(376 MHz, DMSO-D₆) δ=-80.1(s, 3F), -108.8(s, 2F), -122.4 to -121.0(m, 9F), - 125.7(s, 2F).

A fluorescent substance (Alexa Fluor (registered trademark) 647, commercially available from ThermoFisher Scientific) was bonded to the N-terminal of the synthesized tripeptide having a heptadecafluorooctyl group (H-Ala-Asp(C₈F₁₇)-Phe-NH₂).

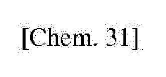

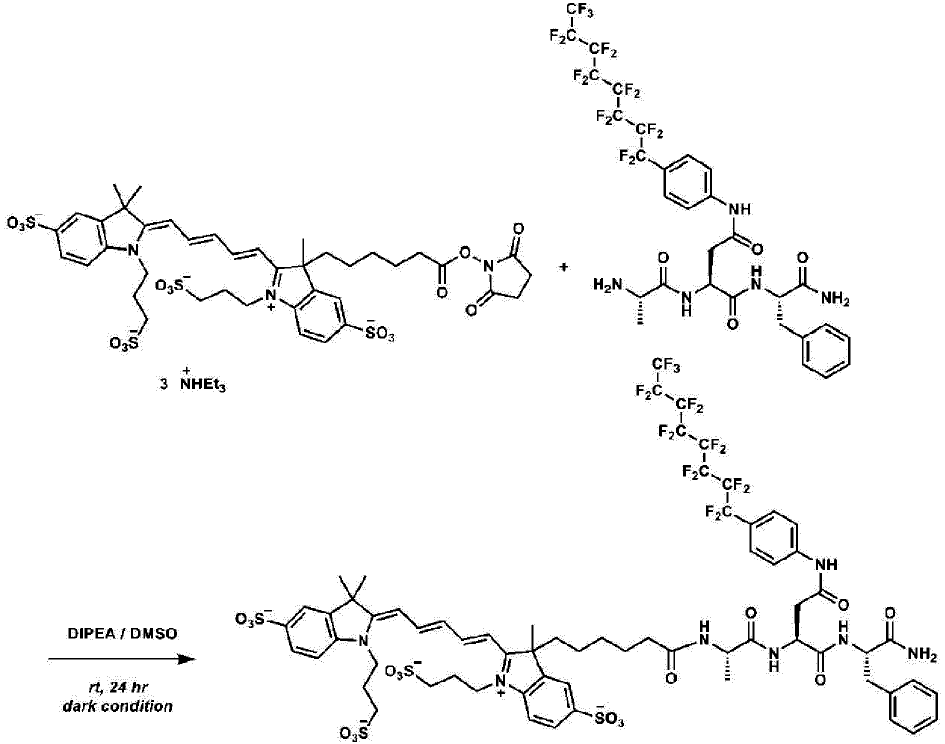

Alexa Fluor647-NHS ester (0.2 mg) dissolved in dry DMSO (20 µL), H-Ala-Asp(C₈F₁₇)-Phe-NH₂ (4.0 equivalents) dissolved in dry DMSO (200 µE), and DIPEA (3.0 equivalents) dissolved in dry DMSO (10 µL) were put into a 1.5 mL black tube. The mixture was continuously stirred at room temperature overnight, then purified through reversed-phase chromatography (acetonitrile/water/TFA=25:75:0.1 to 99:1:0.1), and freeze-dried to obtain a fluorescent conjugate 1 as a blue solid (yield 33% calculated by a fluorometer). Here, the fluorescence was measured using Nano Drop (registered trademark) spectrophotometer ND-1000 at an emission wavelength of 650 nm.

MALDI-TOF MS [M+4H]⁺: m/z calcd for C₆₆H₇₁F₁₇N₇O₁₇S₄₁⁺ 1684. 3512, found 1684.5611

### [Example 2]

### Synthesis of tripeptide H-Ala-Asp(bis-C₄F₉)-Phe-NH₂

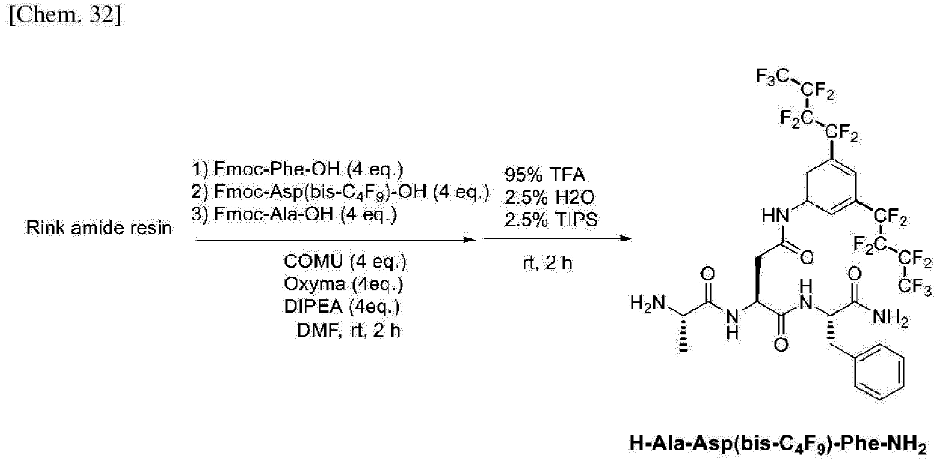

A tripeptide was synthesized by the same method as in Example 1 using Fmoc-Asp(bis-C₄F₉)-OH synthesized in Production Example 3 in place of Fmoc-Asp(C₈F₁₇)-OH for the amino acid to be condensed to obtain H-Ala-Asp(bis-C₄F₉)-Phe-NH₂ (13 mg, 15 µmol, yield 63%).

H-Ala-Asp(bis-C₄F₉)-Phe-NH₂:
LRMS(LC-MS)[M+H]⁺: m/z calcd for C₃₀H₂₆F₁₈N₅O₄^{k} 862. 17, found 862.20

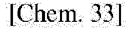

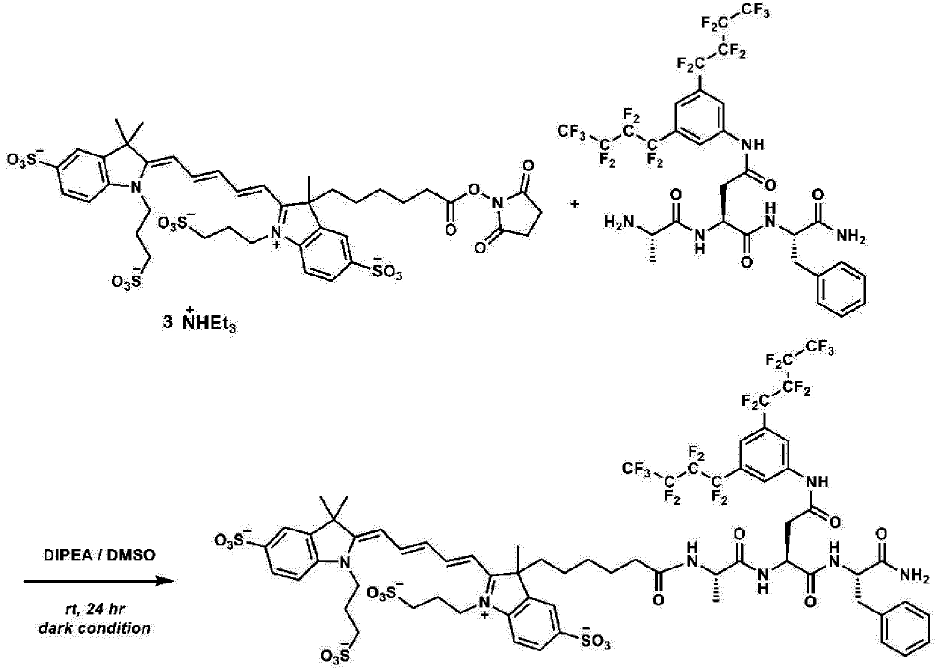

H-Ala-Asp(bis-C₄F₉)-Phe-NH₂ was bound to Alexa Fluor 647 by the same method as in Example 1 to obtain a fluorescent conjugate 2 of H-Ala-Asp(bis-C₄F₉)-Phe-NH₂ as a blue solid (yield 32% calculated by a fluorometer, based on dye).

MALDI-TOF MS[M+3H]: m/z calcd for C₆₆H₆₉F₁₈N₇O₁₇S₄ 1701.3345, found 1701.8410

### [Example 3]

### Synthesis of tripeptide H-Ala-Asp(SF₅)-Phe-NH₂

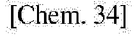

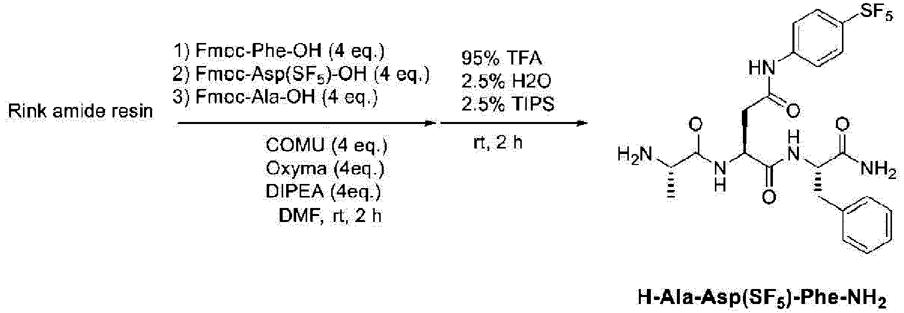

A tripeptide was synthesized by the same method as in Example 1 using Fmoc-Asp(SF₅)-OH synthesized in Production Example 4 in place of Fmoc-Asp(C₈F₁₇)-OH for the amino acid to be condensed to obtain H-Ala-Asp(SF₅)-Phe-NH₂ (9.6 mg, 17 µmol, yield 70%).

H-Ala-Asp(SF₅)-Phe-NH₂
LRMS(LC-MS)[M+H]⁺: m/z calcd for C₂₂H₂₇F₅N₅O₄S⁺ 552.17, found 552.23

### [Example 4]

### Synthesis of tripeptide H-Ala-Asp(tris-t-C₄F₅)-Phe-NH₂

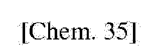

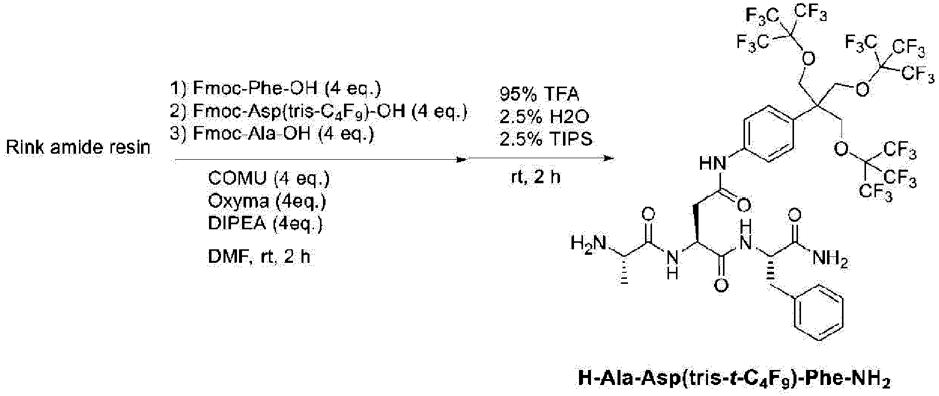

A tripeptide was synthesized by the same method as in Example 1 using Fmoc-Asp(tris-t-C₄F₉)-OH synthesized in Production Example 5 in place of Fmoc-Asp(C₈F₁₇)-OH for the amino acid to be condensed to obtain H-Ala-Asp(tris-t-C₄F₉)-Phe-NH₂ (10 mg, 5 µmol, yield 34%).

H-Ala-Asp(tris-t-C₄F₉)-Phe-NH₂:
LRMS(LC-MS)[M+H]⁺: miz calcd for C₃₈H₃₃F₂₇N₅O₇⁺ 1184.19, found 1184.86

### [Example 5]

### Synthesis of tripeptide H-Ala-Asp(C₄F₉)-Phe-NH₂, H-Ala-Asp(C₆F₁₃)-Phe-NH₂, H-Ala-Asp(C₁₀F₂₁)-Phe-NH₂, H-Ala-Asp(SF₄CF₂CF₂Cl)-Phe-NH₂

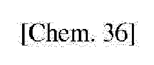

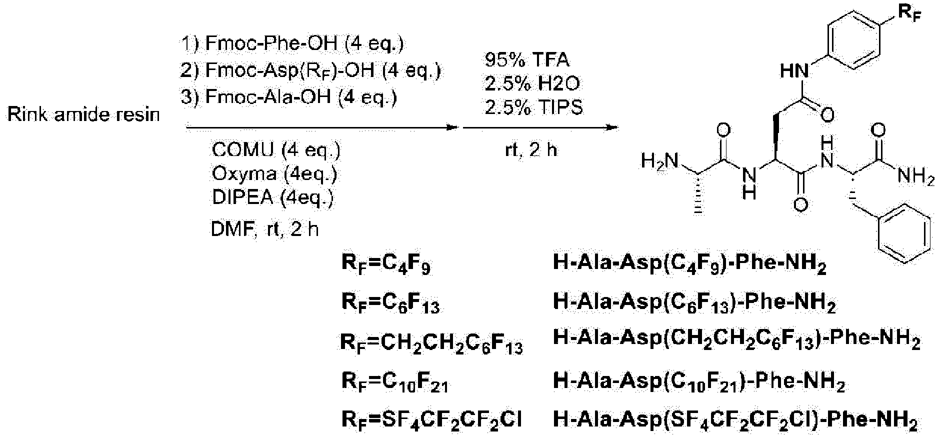

A tripeptide was synthesized by the same method as in Example 1 using Fmoc-Asp(C₄F₉)-OH synthesized in Production Example 7 in place of Fmoc-Asp(C₈F₁₇)-OH for the amino acid to be condensed to obtain H-Ala-Asp(C₄F₉)-Phe-NH₂ (2 mg, 3.1 µmol, yield 12%).

H-Ala-Asp(C₄F₉)-Phe-NH₂:
LRMS(LC-MS)[M+H]⁺: m/z calcd for C₂₆H₃₃F₉N₅O₄⁺ 644.19, found 644.16

In addition, a tripeptide was synthesized by the same method as in Example 1 using Fmoc-Asp(C₆F₁₃)-OH synthesized in Production Example 7 in place of Fmoc-Asp(C₈F₁₇)-OH for the amino acid to be condensed to obtain H-Ala-Asp(C₆F₁₃)-Phe-NH₂ (2.1 mg, 2.8 µmol, yield 11%).

H-Ala-Asp(C₆F₁₃)-Phe-NH₂:
LRMS(LC-MS)[M+H]⁺: m/z calcd for C₃₈H₂₇F₁₃N₅O₄⁺ 744.18, found 744.16

In addition, a tripeptide was synthesized by the same method as in Example 1 using Fmoc-Asp(CH₂CH₂C₆F₁₃)-OH synthesized in Production Example 7 in place of Fmoc-Asp(C₈F₁₇)-OH for the amino acid to be condensed to obtain H-Ala-Asp(CH₂CH₂C₆F₁₃)-Phe-NH₂ (5 mg, 6.4 µmol, yield 25%).

H-Ala-Asp(CH₂CH₂C₆F₁₃)-Phe-NH₂:
LRMS(LC-MS)[M+H]⁺: m/z calcd for C₃₀H₃₁F₁₃N₅O₄⁺ 772.72, found 772.36

In addition, a tripeptide was synthesized by the same method as in Example 1 using Fmoc-Asp(C₁₀F₂₁)-OH in place of Fmoc-Asp(C₈F₁₇)-OH for the amino acid to be condensed to obtain H-Ala-Asp(C₁₀F₂₁)-Phe-NH₂ (1.8 mg, 1.9 µmol, yield 8%). Here, Fmoc-Asp(C₁₀F₂₁)-OH was obtained by subjecting Compound (22a) synthesized in Production Example 7 to a deallylation reaction by the same method as in Production Example 1. The obtained product was used for tripeptide synthesis without purification.

H-Ala-Asp(C₁₀F₂₁)-Phe-NH₂:
LRMS(LC-MS)[M+H]⁺: m/z calcd for C₃₈H₂₇F₁₃N₅O₄⁺ 943.16, found 943.27

In addition, a tripeptide was synthesized by the same method as in Example 1 using Fmoc-Asp(SF₄CF₂CF₂Cl)-OH synthesized in Production Example 7 in place of Fmoc-Asp(C₈F₁₇)-OH for the amino acid to be condensed to obtain H-Ala-Asp(SF₄CF₂CF₂Cl)-Phe-NH₂ (1.8 mg, 2.7 µmol, yield 11%).

H-Ala-Asp(SF₄CF₂CF₂Cl)-Phe-NH₂:
LRMS(LC-MS)[M+H]⁺: m/z calcd for C₂₄H₂₇ClF₈N₅O₄S⁺ 668.13, found 668.14

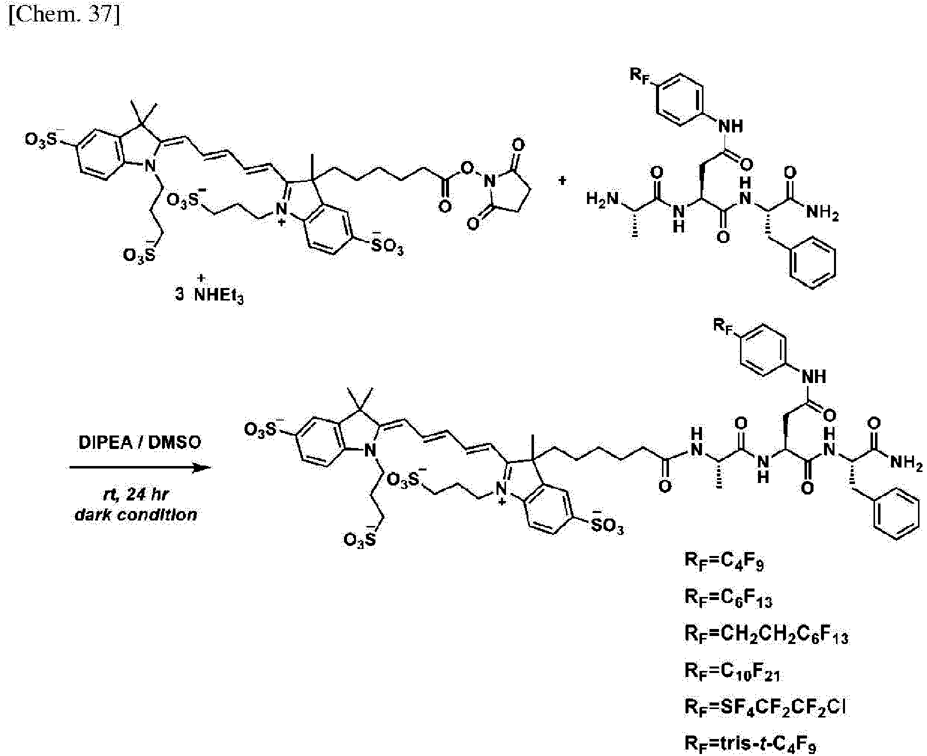

H-Ala-Asp(C₄F₉)-Phe-NH₂ was bound to Alexa Fluor 647 by the same method as in Example 1 to obtain a fluorescent conjugate 6 of H-Ala-Asp(C₄F₉)-Phe-NH₂ as a blue solid (yield 54% calculated by a fluorometer, based on dye).

MALDI-TOF MS [M+3H]: m/z calcd for C₆₆H₆₉F₁₈N₇O₁₇S₄ 1701.3345, found 1701.8410

H-Ala-Asp(C₆F₁₃)-Phe-NH₂ was bound to Alexa Fluor 647 by the same method as in Example 1 to obtain a fluorescent conjugate 7 of H-Ala-Asp(C₆F₁₃)-Phe-NH₂ as a blue solid (yield 50% calculated by a fluorometer, based on dye).

MALDI-TOF MS [M+3H]: m/z calcd for C₆₄H₆₇F₁₃N₇O₁₇S₄ 1583.3503, found 1583.7450

H-Ala-Asp(CH₂CH₂C₆F₁₃)-Phe-NH₂ was bound to Alexa Fluor 647 by the same method as in Example 1 to obtain a fluorescent conjugate 12 of H-Ala-Asp(CH₂CH₂C₆F₁₃)-Phe-NH₂ as a blue solid (yield 96% calculated by a fluorometer, based on dye).

MALDI-TOF MS[M+4H]⁺: m/z calcd for C₆₆H₇₅F₁₃N₇O₁₇S₄⁺ 1612.3889, found 1612.8993

In addition, H-Ala-Asp(C₁₀F₂₁)-Phe-NH₂ was bound to Alexa Fluor 647 by the same method as in Example 1 to obtain a fluorescent conjugate 8 of H-Ala-Asp(C₁₀F₂₁)-Phe-NH₂ as a blue solid (yield 72% calculated by a fluorometer, based on dye).

MALDI-TOF MS [M+4H]: m/z calcd for C₆₈H₇₁F₂₁N₇O₁₇S₄ 1784.32, found 1784.56

In addition, H-Ala-Asp(SF₄CF₂CF₂Cl)-Phe-NH₂ was bound to Alexa Fluor 647 by the same method as in Example 1 to obtain a fluorescent conjugate 9 of H-Ala-Asp(SF₄CF₂CF₂Cl)-Phe-NH₂ as a blue solid (yield 42% calculated by a fluorometer, based on dye).

MALDI-TOF MS[M+3H]: m/z calcd for C₆₀H₇₁ClF₈N₇O₁₇S₅⁺ 1508.3065, found 1508.476

In addition, H-Ala-Asp(tris-t-C₄F₉)-Phe-NH₂ was bound to Alexa Fluor 647 by the same method as in Example 1 to obtain a fluorescent conjugate 10 of H-Ala-Asp(tris-t-C₄F₉)-Phe-NH₂ as a blue solid (yield 89% calculated by a fluorometer, based on dye).

### MALDI-TOF MS[M+3H]: m/z calcd for C₆₀H₇₁ClF₈N₇O₁₇S₅⁺ 1508.3065, found 1508.476

### [Example 6]

### Synthesis of tripeptide H-Ala-Asp(CH₂CH₂C₈F₁₇)-Phe-NH₂

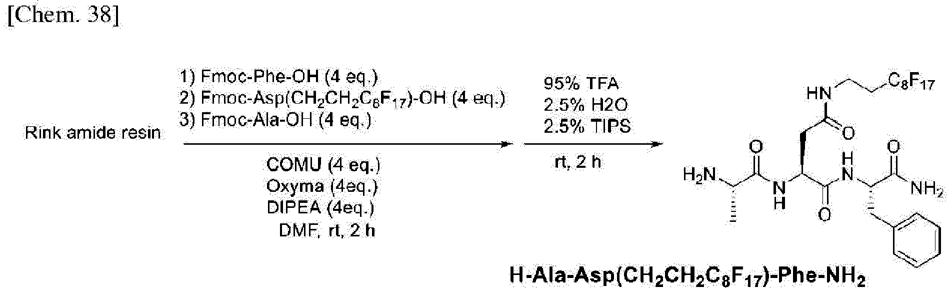

A tripeptide was synthesized by the same method as in Example 1 using Fmoc-Asp(CH₂CH₂C₈F₁₇)-OH synthesized in Production Example 8 in place of Fmoc-Asp(C₈F₁₇)-OH for the amino acid to be condensed to obtain H-Ala-Asp(CH₂CH₂C₈F₁₇)-Phe-NH₂ (5 mg, 6.27 µmol, yield 25%).

### H-Ala-Asp(CH₂CH₂C₈F₁₇)-Phe-NH₂:

LCMS(LC-MS)[M+H]⁺: m/z calcd for C₃₂H₂₆F₁₈N₅O₄⁺ 871.20, found 871.35

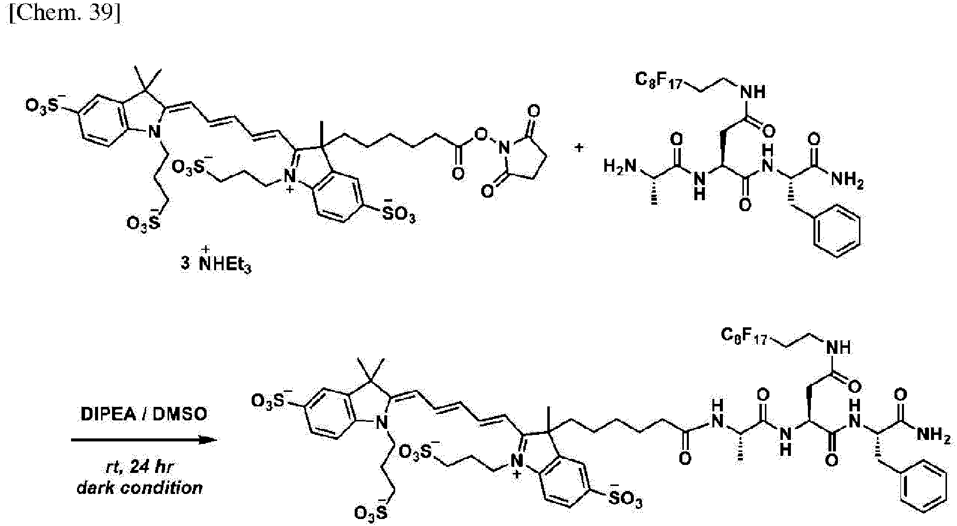

H-Ala-Asp(CH₂CH₂C₈F₁₇)-Phe-NH₂ was bound to Alexa Fluor 647 by the same method as in Example 1 to obtain a fluorescent conjugate 11 of H-Ala-Asp(CH₂CH₂C₈F₁₇)-Phe-NH₂ as a blue solid (yield 80% calculated by a fluorometer, based on dye).

MALDI-TOF MS[M+3H]: m/z calcd for C₆₆H₆₉F₁₈N₇O₁₇S₄ 1708.35, found 1708.82

### [Example 7]

### Synthesis of tetrapeptide H-Cys-Ala-Asp(C₈F₁₇)-Phe-NH₂, H-Cys-Ala-Asp(C₆F₁₃)-Phe-NH₂

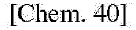

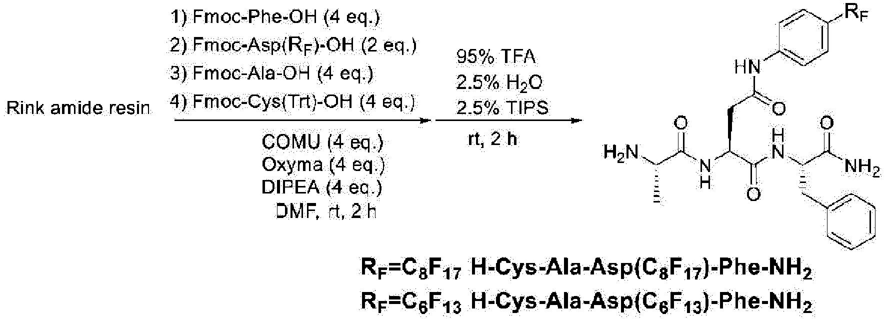

A tetrapeptide was synthesized by the same method as in Example 1 using Fmoc-Asp(C₈F₁₇)-OH for the amino acid to be condensed to obtain H-Cys-Ala-Asp(C₈F₁₇)-Phe-NH₂ (16 mg, 17 µmol, yield 22%).

H-Cys-Ala-Asp(C₈F₁₇)-Phe-NH₂:
LRMS(LC-MS)[M+H]⁺: m/z calcd for C₃₃H₃₂F₁₇N₆O₅S⁺ 947.69, found 947.54

In addition, a tetrapeptide was synthesized by the same method as in Example 1 using Fmoc-Asp(C₆F₁₃)-OH synthesized in Production Example 7 in place of Fmoc-Asp(C₈F₁₇)-OH for the amino acid to be condensed to obtain H-Cys-Ala-Asp(C₆F₁₃)-Phe-NH₂ (12 mg, 15 µmol, yield 57%).

H-Cys-Ala-Asp(C₆F₁₃)-Phe-NH₂:
LRMS(LC-MS)[M+H]⁺: m/z calcd for C₃₁H₃₂Fi₃N₆O₅S⁺ 847.67, found 847.54

### [Comparative Example 1]

### Synthesis of tripeptide H-Ala-Asp(C₁₂H₂₅)-Phe-NH₂

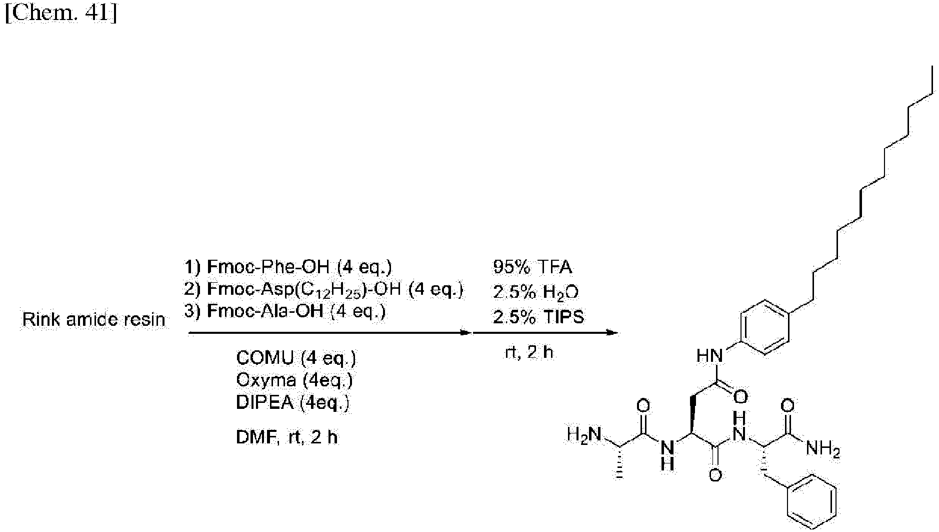

A tripeptide was synthesized by the same method as in Example 1 using Fmoc-Asp(C₁₂H₂₅)-OH containing no fluorine atom synthesized in Production Example 6 in place of Fmoc-Asp(C₈F₁₇)-OH for the amino acid to be condensed to obtain H-Ala-Asp(C₁₂H₂₅)-Phe-NH₂ (7.1 mg, 11 µmol, yield 48%).

H-Ala-Asp(C₁₂H₂₅)-Phe-NH₂:
1H-NMR(500 MHz, DMSO-D₆)δ 9.86(s, 1H), 7.04(m, 4H), 4.52(m, 1H), 4.34(m, 1H), 4.09(m, 1H), 2.89-3.05(m, 2H), 2.65-2.81(m, 2H), 1.23-1.98(m, 28H).

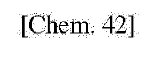

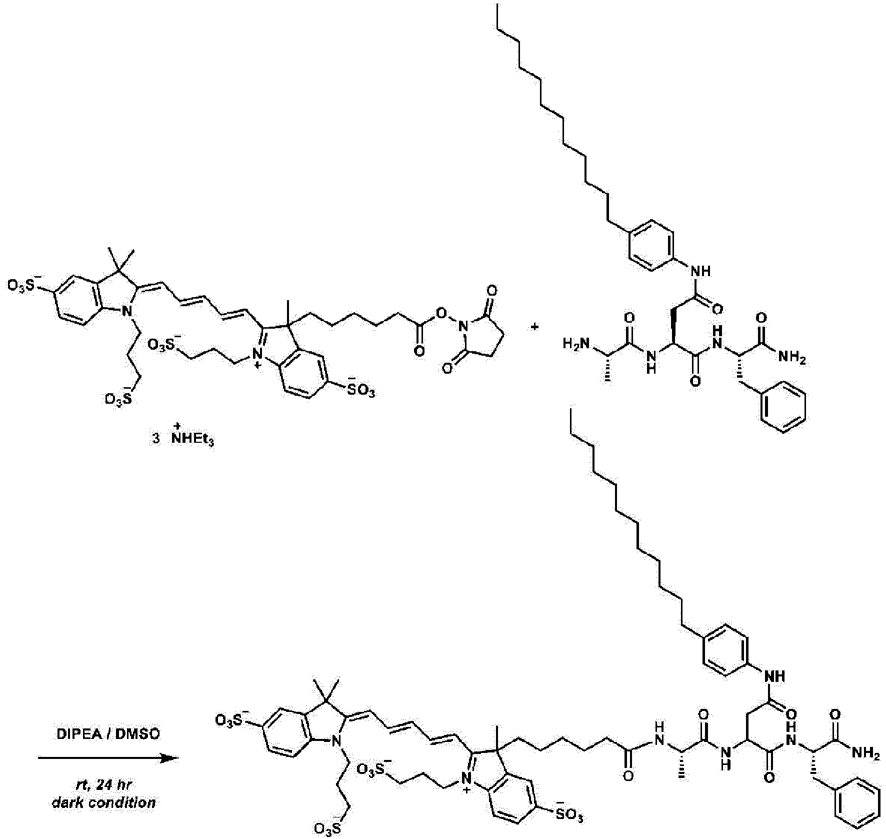

H-Ala-Asp(C₁₂H₂₅)-Phe-NH₂ was bound to Alexa Fluor 647 by the same method as in Example 1 to obtain a fluorescent conjugate 3 of H-Ala-Asp(C₁₂H₂₅)-Phe-NH₂ as a blue solid (yield 23% calculated by a fluorometer, based on dye).

MALDI-TOF MS [M+4H]⁺: m/z calcd for C₇₀H₉₆N₇O₁₇S₄⁺ 1434, 5740, found 1434.1390

### [Comparative Example 2]

A dipeptide (Boc-RFAA(C8)-Phe-OMe) having a heptadecafluorooctyl group was synthesized.

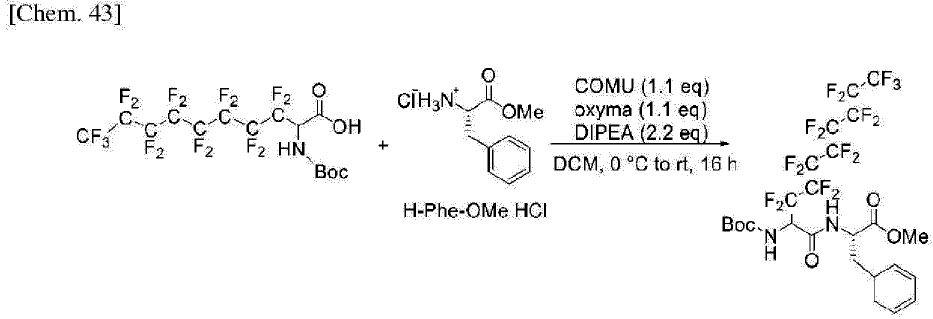

In a dry 300 mL three-necked eggplant flask, 2-((tert-butoxycarbonyl)amino)-3,3,4,4,5,5,6,6,7,7,8,8,9,9,10,10,10-heptadecafluorododecanoic acid (994.4 mg, 1.68 mmol), DCM (99 mL), phenylalanine methyl ester hydrochloride (1.84 mmol), and oxyma (1.84 mmol) were mixed and stirred. The reaction mixture was cooled to 0°C, COMU (1.84 mmol) and DIPEA (3.69 mmol) were added, and the mixture was stirred at room temperature for 20.5 hours. Then, the reaction mixture was quenched with HCl (IN) and extracted three times with DCM. The combined organic phase was concentrated under a reduced pressure and then diluted with ethyl acetate, and washed with HCl (1N), a saturated sodium bicarbonate solution, and a saturated saline solution. The washed organic phase was dried with sodium sulfate, filtered, and then concentrated under a reduced pressure to obtain a crude product of Boc-RFAA(C8)-Phe-OMe. The crude product was purified through silica gel chromatography (ethyl acetate/hexane=1/4 (volume ratio)) to obtain a total amount of 807.3 mg (1.07 mmol, yield 63.8%) of Boc-[(R)-RFAA(C8)]-Phe-OMe (diastereomer A) (383.3 mg, 0.508 mmol), and Boc-[(S)-RFAA(C8)]-Phe-OMe (diastereomer B) (417.1 mg, 0.553 mmol).

Here, using crystals of the diastereomer B, the three-dimensional structure of RFAA(C8) contained was determined to be the (S) form by X-ray structural analysis. For X-ray structural analysis, VariMax Dual Saturn (commercially available from Rigaku Corporation) was used.

Boc-[(R)-RFAA(C8)]-Phe-OMe (diastereomer A):
¹H NMR(500 MHz, CDCl₃) δ=7.30-7.25(m, 3H), 7.07-7.05(m, 2H), 6.40(d, 1H), 5.46(m, 1H), 5.00-4.87(m, 2H), 3.77(s, 3H), 3.20-3.11(m, 2H), 1.47(s, 9H).
¹⁹F NMR(470 MHz, CDCl₃) δ=-80.86(t, 3F), -114.89(d, J=281 Hz, 1F), -119.11(d, J=279 Hz, 1F), -120.40 to -123.10(m, 10F), -126.23(m, 2F).
Boc-RFAA(C8)-Phe-OMe

Boc-[(S)-RFAA(C8)]-Phe-OMe (diastereomer B):
¹H NMR(500 MHz, CDCl₃) δ=7.28-7.26(m, 3H), 7.10-7.04(m, 2H), 6.35(d, 1H), 5.48(m, 1H), 5.00-4.87(m, 2H), 3.74(s, 3H), 3.15-3.13(m, 2H), 1.47(s, 9H).
¹⁹F NMR(470 MHz, CDCl₃) -80.86(t, 3F), -114.21(d, J=275 Hz, 1F), -118.78(d, J=279 Hz, 1F), -120.35 to -123.00(m, 10F), -126.23(m, 2F).

The protecting group on the side of the N-terminal of the synthesized peptide was deprotected to obtain H-RFAA(C8)-Phe-OMe.

A stirring bar was put into a 50 mL two-necked flask, and Boc-[(R)-RFAA(C8)]-Phe-OMe (diastereomer A) (0.508 mmol) and DCM (17 mL) were added. The reaction mixture was cooled to 0°C, TFA (3.4 mL) was then added, and the temperature was raised to room temperature. After stirring for 1.5 hours, a sodium bicarbonate aqueous solution was added, and the reaction was terminated. The aqueous phase was extracted with DCM, and the combined organic phase was distilled off under a reduced pressure to obtain a crude product of a deprotected form H-[(R)-RFAA(C8)]-Phe-OMe. The crude product was purified through silica gel chromatography (ethyl acetate/hexane=2/5 (volume ratio)) to obtain H-[(R)-RFAA(C8)]-Phe-OMe (diastereomer A) (0.393 mmol, yield 67.7%).

H-[(R)-RFAA(C8)]-Phe-OMe (diastereomer A)
⁴1 NMR(500 MHz, ACETONE-D6) δ=7.95(br, 1H), 7.30-7.22(m, 5H), 4.82-4.77(m, 1H), 4.40-4.25(m, 1H), 3.67(s, 3H), 3.20-3.07(m, 2H).
¹⁹F NMR(470 MHz, ACETONE-D6) δ=-80.86(t, 3F), -115.00(d, J=277 Hz, 1F), -117.20(d, J=275 Hz, 1F), -120.00 to -122.70(m, 10F), -125.95(s, 2F).

Boc-[(S)-RFAA(C8)]-Phe-OMe (diastereomer B) (98.7 µmol) was deprotected by the same method to obtain H-[(S)-RFAA(C8)]-Phe-OMe (diastereomer B) (60.1 µmol, yield 60.8%).

H-[(S)-RFAA(C8)]-Phe-OMe (diastereomer B):
¹H NMR(500 MHz, ACETONE-D6) δ=7.31-7.19(m, 5H), 4.82-4.79(m, 1H), 4.70-4.64(m, 1H), 3.72(s, 3H), 3.22-3.06(m, 2H).
¹⁹F NMR(470MHz, ACETONE-D6) δ=-80.86(t, 3F), -114.76(d, J=267 Hz, 1F), -117.31(d, J=275 Hz, 1F), -120.00 to -122.70(m, 10F), -125.94(s, 2F).

Subsequently, a tripeptide (Boc-Ala-RFAA(C8)-Phe-OMe) having a heptadecafluorooctyl group was synthesized.

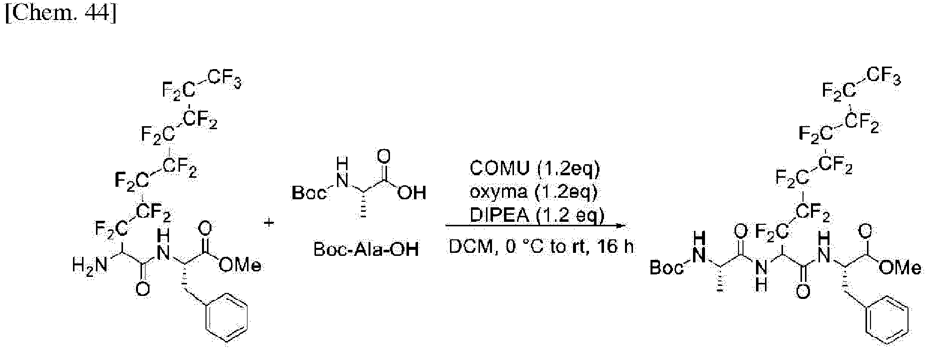

In a 25 mL two-necked round bottom flask, dipeptide (H-[(R)-REAA(C8)]-Phe-OMe) (0.24 mmol), Boc-Ala-OH (1.2 equivalents), and oxyma (1.2 equivalents) were dissolved in DCM (3.4 mL) and stirred. The reaction mixture was cooled to 0°C, COMU (1.2 equivalents) and DIPEA (1.2 equivalents) were added, and the mixture was stirred at room temperature for 24 hours. Then, the reaction mixture was quenched with HCl (1N) and extracted three times with DCM. The combined organic phase was concentrated under a reduced pressure and then diluted with ethyl acetate, and washed with HCl (1N), a saturated sodium bicarbonate solution, and a saturated saline solution. The washed organic phase was dried with sodium sulfate, filtered, and then concentrated under a reduced pressure to obtain a crude product of Boc-Ala-[(R)-RFAA(C8)]-Phe-OMe. The crude product was dissolved in DCM and then reprecipitated with hexane, and filtered to obtain pure Boc-Ala-[(R)-RFAA(C8)]-Phe-OMe (0.20 mmol, yield 85.4%).

Boc-Ala-[(R)-RFAA(C8)]-Phe-OMe (diastereomer A):
¹H NMR(500 MHz, ACETONE-D6) δ=8.29(br, 1H), 7.72(br, 1H), 7.28-7.17(m, 5H), 6.38(br, 1H), 5.67-5.60(m, 1H), 4.80-4.76(m, 1H), 4.23-4.18(m, 1H), 3.67(s, 3H), 3.20-3.00(m, 2H), 1.43(s, 9H)1.30(t, 3H).
¹⁹F NMR(470MHz, ACETONE-D6) δ=-80.86(t, 3F), -114.57(d, J=282 Hz, 1F), -119.31(d, J=281 Hz, 1F), -120.00 to -122.70(m, 10F), -125.96(m, 2F).

H-[(S)-RFAA(C8)]-Phe-OMe (diastereomer B) (46.1 µmol) was condensed with Boc-Ala-OH by the same method to obtain Boc-Ala-[(S)-RFAA(C8)]-Phe-OMe (diastereomer B) (45.7 µmol, yield 99%).

Boc-Ala-[(S)-RFAA(C8)]-Phe-OMe (diastereomer B):
¹H NMR(500 MHz, ACETONE-D6) δ=8.33(br, 1H), 7.85(br, 1H), 7.30-7.22(m, 5H), 6.30(br, 1H), 5.63-5.53(m, 1H), 4.72-4.67(m, 1H), 4.32-4.25(m, 1H), 3.66(s, 3H), 3.18-3.02(m, 2H), 1.40(s, 9H)1.32(d, 3H).
¹⁹F NMR(470 MHz, ACETONE-D6) δ=-80.86(t, 3F), -114.50(d, J=287 Hz, 1F), -118.77(d, J=277 Hz, 1F), -120.75 to -122.53 (m, 10F), -125.93(m, 2F).

The protecting group on the side of the N-terminal of the synthesized peptide was deprotected to obtain H-Ala-[(R)-RFAA(C8)]-Phe-OMe.

A stirring bar was put into a 20 mL two-necked flask, and Boc-Ala-[(R)-RFAA(C8)]-Phe-OMe (diastereomer A) (0.194 mmol) and DCM (5 mL) were added to one side. The reaction mixture was cooled to 0°C, TFA (1 mL) was then added, the temperature was raised to room temperature, and stirring was performed for 2 hours. Then, a sodium bicarbonate aqueous solution was added and the reaction was terminated. The aqueous phase of the reaction mixture was extracted with DCM, and the combined organic phase was distilled off under a reduced pressure to obtain a crude product of a deprotected form H-Ala-[(R)-RFAA(C8)]-Phe-OMe. The crude product was purified through silica gel chromatography (chloroform/methanol=15/1 (volume ratio)) to obtain H-Ala-[(R)-RFAA(C8)]-Phe-OMe (diastereomer A) (0.147 mmol, yield 75.6%).

H-Ala-[(R)-RFAA(C8)]-Phe-OMe (diastereomer A):
¹H NMR(500 MHz, ACETONE-D6) δ=8.30, 8.20(m, NH), 7.28-7.19(m, 5H), 6.38(br, 1H), 5.68-5.53(m, 1H), 4.79-4.76(m, 1H), 3.97-3.47(m, 1H), 3.67(s, 3H), 1.26, 1.17(s, 3H).
¹⁹F NMR(470MHz, ACETONE-D6) δ=-80.86(t, 3F), -114.50(d, J=282 Hz, 1F), -119.48(d, J=281 Hz, 1F), -120.40 to -122.65(m, 10F), -125.92(m, 2F).

Boc-Ala-[(S)-RFAA(C8)]-Phe-OMe (diastereomer B) (45.7 µmol) was deprotected by the same method to obtain H-Ala-[(S)-RFAA(C8)]-Phe-OMe (diastereomer B) (21.1 µmol, yield 46.2%).

H-Ala-[(S)-RFAA(C8)]-Phe-OMe (diastereomer B):
¹H NMR(500 MHz, ACETONE-D6) δ=8.38, 8.21(m, NH), 7.31-7.21(m, 5H), 5.59-5.54(m, 1H), 4.76-4.72(m, 1H), 4.06-3.97(m, 1H), 3.67(s, 3H), 3.20-3.04(m, 2H), 1.19, 1.17(s, 3H).
¹⁹F NMR(470 MHz, ACETONE-D6) δ=-80.86(t, 3F), -114.82(d, J=282 Hz, 1F), -118.68(d, J=284 Hz, 1F), -120.80 to -122.70(m, 10F), -125.98(m, 2F).

A fluorescent substance Alexa Fluor647 was bound to the N-terminal of the synthesized tripeptide after deprotection (H-Ala-[(R)-RFAA(C8)]-Phe-OMe).

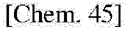

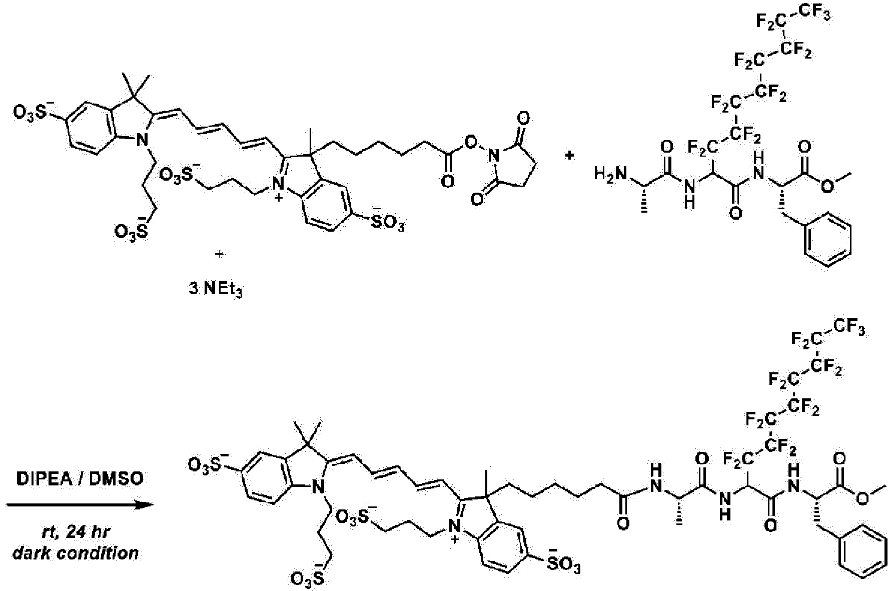

Alexa Fluor 647-NHS ester (1.75 mg) dissolved in dry DMSO (175 µL), H-Ala-RFAA(C8)-Phe-OMe (3.0 equivalents) dissolved in dry DMSO (50 µL), DIPEA (3.0 equivalents) dissolved in dry DMSO (71 µL) and dry DMSO 124 µL were put into a 1.5 mL black tube. The mixture was continuously stirred at room temperature overnight. The mixture was purified through reversed-phase chromatography (acetonitrile/water/TFA=25:75:0.1 to 99:1:0.1), and freeze-dried to obtain a fluorescent conjugate 4 as a blue solid (yield 52.0% calculated by a fluorometer). Here, the fluorescence was measured using Nano Drop (registered trademark) spectrophotometer ND-1000 at an emission wavelength of 650 nm.

### MALDI-TOF MS

[M+2]⁻: m/z calcd for C₅₉H₆₃F₁₇N₅O₁₇S₄-1564.2825, found 1564.3501

H-Ala-[(S)-RFAA(C8)]-Phe-OMe (diastereomer B) (1.241 µmol) was bound to Alexa Fluor^{™} 647 by the same method to obtain a fluorescent conjugate 5 of H-Ala-[(S)-RFAA(C8)]-Phe-OMe (diastereomer B) as a blue solid (yield 54.3% calculated by a fluorometer, based on dye).

### MALDI-TOF MS

[M+4]⁺: m/z calcd for C₅₉H₆₅F₁₇N₅O₁₇S₄+1566.3935, found 1566.3679

### [Test Example 1]

### Comparison of peptide fluorescent conjugates 1, 4, and 5 and fluorescent dye 1

The efficiencies of uptake into cells of the peptide fluorescent conjugate 1 synthesized in Example 1 (Alexa-Ala-Asp(C₈F₁₇)-Phe-NH₂), the peptide fluorescent conjugate 4 synthesized in Comparative Example 2 (Alexa-Ala-[(R)-RFAA(C8)]-Phe-OMe) and the peptide fluorescent conjugate 5 (Alexa-Ala-[(S)-RFAA(C8)]-Phe-OMe), and the diethylamide form of the fluorescent substance Alexa Fluor647 (fluorescent dye 1) were examined.

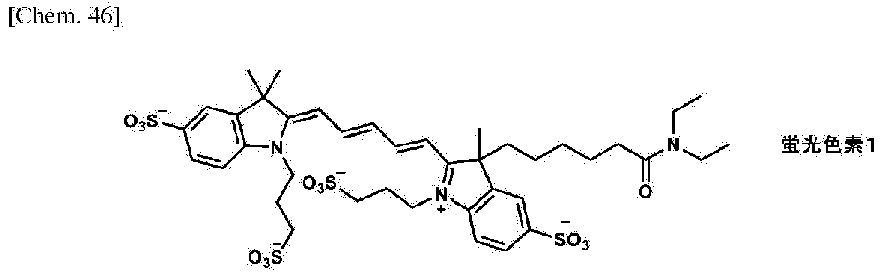

### (Preparation of sample solution)

A DMSO solution of the synthesized peptide fluorescent conjugates 1, 4, and 5, and fluorescent dye 1 was diluted using a DMEM low-glucose culture solution to a concentration of 1.5 µM, the concentration of the DMSO contained was adjusted to 0.15%, and the resulting solution was used as a sample solution for a cell membrane permeability test.

### (Evaluation of ability to permeate cell membrane)

HeLa cells were seeded in a 96-well cell culture plate 24 hours before peptide treatment (0.5×10⁵ cells/well).

The medium of HeLa cells pre-cultured at 37°C for 24 hours was replaced with a sample solution of the peptide fluorescent conjugates 1, 4, and 5 and the fluorescent dye 1, and the ability to permeate a cell membrane after culturing at 37°C for 1 hour was evaluated. After culturing for each predetermined time, the cell surface was washed three times with phosphate-buffered saline (PBS), and the cells were detached and collected with Trypsin-EDTA 0.05% (commercially available from Gibco). The collected cells were analyzed by flow cytometry (guava easyCyte (trademark) 8) measuring red 2 fluorescence (661/15 nm) for detecting the fluorescent dye (Alexa Fluor647) introduced into the peptide fluorescent conjugate. FIG. 1 shows the flow cytometry analysis results of cells cultured in the sample solution at 37°C for 1 hour. The vertical axis represents the number of cells (count), and the horizontal axis represents the fluorescence intensity of the cells. In addition, FIG. 2 shows the comparison results of the mean fluorescence intensity (MFI) of the samples. Here, in the drawings, PFCJ indicates a peptide fluorescent conjugate, and FD indicates a fluorescent dye.

As shown in FIG. 1, regarding the fluorescence intensity of Alexa Fluor 647 in cells after incubation at 37°C for 1 hour, the cells treated with the peptide fluorescent conjugates 1, 4, and 5 having a fluoroalkyl group in the side chain all had a higher fluorescence intensity than the cells treated with the fluorescent dye 1. As shown in FIG. 2, the average value of the fluorescence intensities of Alexa Fluor 647 in cells was about several times greater for the cells treated with the peptide fluorescent conjugate 4 and the peptide fluorescent conjugate 5 in which a fluoroalkyl group was directly bonded to the α carbon of the peptide main chain, and several tens to several hundreds of times greater for the cells treated with the peptide fluorescent conjugate 1 in which a fluoroalkyl group was introduced into the carboxy group of the side chain of aspartic acid via a peptide bond and a phenylene group, than for the cells treated with the fluorescent dye 1. Based on these results, it was found that peptides having a fluoroalkyl group in the side chain had higher efficiency of uptake into cells and a better ability to permeate a cell membrane than commercially available cell-penetrating peptides and fluorescent dyes; particularly, peptides in which a fluoroalkyl group was linked to the α carbon via a linking group had a better ability to permeate a cell membrane than peptides in which the side chain itself bonded to the α carbon of the peptide main chain was a fluoroalkyl group.

### [Test Example 2]

### Comparison of peptide fluorescent conjugates 1 and 2, TAT-Alexa, and fluorescent dye 1

The efficiencies of uptake into cells of the peptide fluorescent conjugate 1 synthesized in Example 1 (Alexa-Ala-Asp(C₈F₁₇)-Phe-NH₂), the peptide fluorescent conjugate 2 synthesized in Example 2 (Alexa-Ala-Asp(bis-C₄F₉)-Phe-NH₂), the diethylamide form of the fluorescent substance Alexa Fluor 647 (fluorescent dye 1), and the fluorescent substance Alexa Fluor 647 C2 Maleimide added to the terminal Cys of Cys-TAT(47-57)(H-Cys-Tyr-Gly-Arg-Lys-Lys-Arg-Arg-Gln-Arg-Arg-Arg-NH₂) (commercially available cell-penetrating peptide ANA Spec) (TAT-Alexa) were examined.

Using sample solutions of the peptide fluorescent conjugates 1 and 2, TAT-Alexa, and the fluorescent dye 1 prepared in the same manner as in Test Example 1, the red 2 fluorescence (661/15 nm) was measured and analyzed by flow cytometry by the same procedure as in Test Example 1.

FIG. 3 shows the flow cytometry analysis results of cells cultured in the sample solution at 37°C for 1 hour. In addition, FIG. 4 shows the comparison results of the mean fluorescence intensity (MFI) of the samples. In the drawings, PFCJ indicates a peptide fluorescent conjugate, and FD indicates a fluorescent dye.

As shown in FIG. 3, regarding the fluorescence intensity of Alexa Fluor 647 in cells after incubation at 37°C for 1 hour, the cells treated with the peptide fluorescent conjugates 1 and 2 in which a fluoroalkyl group was linked to the side chain via a linking group all had a higher fluorescence intensity than the cells treated with the fluorescent dye 1 or TAT-Alexa. As shown in FIG. 4, it was found that the average value of the fluorescence intensities of Alexa Fluor 647 in cells was about double for the cells treated with TAT-Alexa, and several tens to several hundreds of times greater for the cells treated with the peptide fluorescent conjugates 1 and 2, than for the cells treated with the fluorescent dye 1. Based on these results, it was found that peptides in which a fluoroalkyl group was linked to the side chain via a linking group had higher efficiency of uptake into cells and a better ability to permeate a cell membrane than commercially available cell-penetrating peptides and fluorescent dyes.

### [Test Example 3]

### Comparison of peptide fluorescent conjugates 1 and 3, and fluorescent dye 1 (comparison in the absence of serum and in the presence of serum)

The efficiencies of uptake into cells of the peptide fluorescent conjugate 1 containing a fluorine atom in the side chain synthesized in Example 1 (Alexa-Ala-Asp(C₈F₁₇)-Phe-NH₂), the peptide fluorescent conjugate 3 containing no fluorine atom in the side chain synthesized in Comparative Example 1 (Alexa-Ala-Asp(C₁₂H₂₅)-Phe-NH₂), and the fluorescent dye 1 were examined.

Here, to mimic conditions more similar to those in living organisms, samples containing fetal bovine serum (FBS) were prepared and implemented.

### (Cell membrane permeability test in the absence of FBS)

Using sample solutions of the peptide fluorescent conjugate 1, the peptide fluorescent conjugate 3, and the fluorescent dye 1 prepared in the same manner as in Test Example 1, the red 2 fluorescence (661/15 nm) was measured and analyzed by flow cytometry by the same procedure as in Test Example 1.

FIG. 5 shows the results of comparing the mean fluorescence intensity (MFI) of the samples with respect to the flow cytometry analysis results of cells cultured in a sample solution at 37°C for 1 hour. In the drawings, PFCJ indicates a peptide fluorescent conjugate, and FD indicates a fluorescent dye.

### (Preparation of sample solution containing FBS)

A DMSO solution of the synthesized peptide fluorescent conjugate 1, peptide fluorescent conjugate 3, and fluorescent dye 1 was diluted using a DMEM low-glucose culture solution containing FBS 10% (a DMEM low-glucose medium containing 10% FBS and 1% penicillin-streptomycin solution) to a concentration of 1.5 µM, and the resulting solution was used as a sample solution for a cell membrane permeability test in the presence of FBS.

### (Cell membrane permeability test in the presence of FBS)

Using sample solutions of the peptide fluorescent conjugate 1, the peptide fluorescent conjugate 3, and the fluorescent dye 1 prepared in the same manner as in Test Example 1, the red 2 fluorescence (661/15 nm) was measured and analyzed by flow cytometry by the same procedure as in Test Example 1 except that the sample solution contained FBS.

FIG. 6 shows the results of comparing the mean fluorescence intensity (MFI) of the samples with respect to the flow cytometry analysis result of cells cultured in a sample solution containing FBS at 37°C for 1 hour. In the drawings, PFCJ indicates a peptide fluorescent conjugate, and FD indicates a fluorescent dye.

Regarding the mean fluorescence intensity (MFI) of Alexa Fluor 647 in cells after incubation at 37°C for 1 hour, as shown in FIG. 5, in the test using cells in the absence of FBS, the mean fluorescence intensity was higher in the cells treated with the peptide fluorescent conjugate 3 containing no fluorine atom in the side chain than the cells treated with the peptide fluorescent conjugate 1 containing a fluorine atom in the side chain. On the other hand, in the test using cells in the presence of FBS, as shown in FIG. 6, the MFI value was higher for the peptide fluorescent conjugate 1 containing a fluorine atom in the side chain.

Based on these results, it was found that the fluorine-containing peptide according to the present invention exhibited a high ability to permeate a cell membrane under conditions more similar to those in living organisms.

### [Test Example 4]

### Comparison of peptide fluorescent conjugates 1 to 3, 6, 7, 9, and 10, and fluorescent dye 1

The efficiencies of uptake into cells of the peptide fluorescent conjugate 1 synthesized in Example 1 (Alexa-Ala-Asp(C₈F₁₇)-Phe-NH₂), the peptide fluorescent conjugate 2 synthesized in Example 2 (Alexa-Ala-Asp(bis-C₄F₉)-Phe-NH₂), the peptide fluorescent conjugate 6 synthesized in Example 5 (Alexa-Ala-Asp(C₄F₉)-Phe-NH₂), the peptide fluorescent conjugate 9 synthesized in Example 5 (Alexa-Ala-Asp(SF₄CF₂CF₂Cl)-Phe-NH₂), the peptide fluorescent conjugate 7 synthesized in Example 5 (Alexa-Ala-Asp(C₆F₁₃)-Phe-NH₂), the peptide fluorescent conjugate 10 synthesized in Example 5 (Alexa-Ala-Asp(tris-t-C₄F₉)-Phe-NH₂), the peptide fluorescent conjugate 3 containing no fluorine atom in the side chain (Alexa-Ala-Asp(C₁₂H₂₅)-Phe-NH₂) synthesized in Comparative Example 1, and the diethylamide form of the fluorescent substance Alexa Fluor647 (fluorescent dye 1) were examined.

Using sample solutions of the peptide fluorescent conjugates 1 to 3, 6, 7, 9, and 10, and the fluorescent dye 1 prepared in the same manner as in Test Example 1, the red 2 fluorescence (661/15 nm) was measured and analyzed by flow cytometry by the same procedure as in Test Example 1.

FIG. 7 shows the flow cytometry analysis results of cells cultured in the sample solution at 37°C for 1 hour. In addition, FIG. 8 shows the results of comparing the relative fluorescence intensity (RFI) of the samples based on the fluorescent dye 1. In the drawings, PFCJ indicates a peptide fluorescent conjugate, and FD indicates a fluorescent dye.

As shown in FIGS. 7 and 8, the average value of the fluorescence intensities of Alexa Fluor 647 in cells was higher in the cells treated with the peptide fluorescent conjugates than the cells treated with the fluorescent dye 1. In addition, it can be clearly understood that, when the chain length of the fluoroalkyl group introduced into the peptide was longer, the average value of the fluorescence intensities was higher, and the ability to permeate a cell membrane tended to be stronger.

### [Test Example 5]

### Comparison of particle sizes of peptide fluorescent conjugates 1 to 3, 6, 7, 9, and 10

Using sample solutions of the peptide fluorescent conjugates 1 to 3, 6, 7, 9, and 10 prepared in the same manner as in Test Example 4, the particle size was measured by the dynamic light scattering method.

The concentration of the peptide conjugate was adjusted to 1.5 µM in pure water, 12 µL of the peptide conjugate was put into a quartz cell, the particle size at 37°C was measured using a dynamic light scattering device DLS (Zeta sizer µV 60 mW 830 nm laser), and the particle size distribution was determined. FIG. 9 shows the analysis results of the average particle size (average diameter of number-based distribution) of the peptide fluorescent conjugates. In the drawings, PFCJ indicates a peptide fluorescent conjugate, and FD indicates a fluorescent dye.

Comparing FIG. 8 and FIG. 9, it can be clearly understood that, when the chain length of the fluoroalkyl group introduced into the peptide was longer, the average particle size tended to be smaller. The peptide fluorescent conjugate 1, which was particularly effective at permeating a cell membrane, had a very small average particle size of about 6 nm. The reason the chain length of the fluoroalkyl group affects the average particle size is not clear, but it is speculated that, when the chain length is longer, the aggregation force between the fluoroalkyl groups becomes stronger, and the particle size of all peptide fluorescent conjugates decreases due to this aggregation force.

### [Test Example 6]

### Comparison of peptide fluorescent conjugates 1, 8, and 11, and fluorescent dye 1

The efficiencies of uptake into cells of the peptide fluorescent conjugate 1 synthesized in Example 1 (Alexa-Ala-Asp(C₈F₁₇)-Phe-NH₂), the peptide fluorescent conjugate 8 synthesized in Example 5 (Alexa-Ala-Asp(C₁₀F₂₁)-Phe-NH₂), the peptide fluorescent conjugate 11 synthesized in Example 6 (Alexa-Ala-Asp(CH₂CH₂C₈F₁₇)-Phe-NH₂), and the diethylamide form of the fluorescent substance Alexa Fluor647 (fluorescent dye 1) were examined.

Using sample solutions of the peptide fluorescent conjugates 1, 8, and 11, and the fluorescent dye 1 prepared in the same manner as in Test Example 1, the red 2 fluorescence (661/15 nm) was measured and analyzed by flow cytometry by the same procedure as in Test Example 1.
FIG. 10 shows the flow cytometry analysis results of cells cultured in the sample solution at 37°C for 1 hour. In addition, FIG. 11 shows the comparison results of the mean fluorescence intensity (MFI) of the samples. In the drawings, PFCJ indicates a peptide fluorescent conjugate, and FD indicates a fluorescent dye.

As shown in FIGS. 10 and 11, the average value of the fluorescence intensities of Alexa Fluor 647 in cells was higher in the cells treated with the peptide fluorescent conjugate than the cells treated with the fluorescent dye 1. In addition, it can be clearly understood that, when the chain length of the fluoroalkyl group introduced into the peptide was longer, the average value of the fluorescence intensities was higher, and the ability to permeate a cell membrane tended to be stronger.

### [Test Example 7]

### Comparison of peptide fluorescent conjugates 1, and 12, and fluorescent dye 1

The efficiencies of uptake into cells of the peptide fluorescent conjugate 1 synthesized in Example 1 (Alexa-Ala-Asp(C₈F₁₇)-Phe-NH₂), the peptide fluorescent conjugate 12 synthesized in Example 5 (Alexa-Ala-Asp(CH₂CH₂C₆F₁₃)-Phe-NH₂), and the diethylamide form of the fluorescent substance Alexa Fluor 647 (fluorescent dye 1) were examined.

Using sample solutions of the peptide fluorescent conjugates 1 and 12, or the fluorescent dye 1 prepared in the same manner as in Test Example 1, the red 2 fluorescence (661/15 nm) was measured and analyzed by flow cytometry by the same procedure as in Test Example 1.

FIG. 12 shows the comparison results of the mean fluorescence intensity (MFI) of the samples. In the drawings, PFCJ indicates a peptide fluorescent conjugate, and FD indicates a fluorescent dye.

As shown in FIG. 12, regarding the fluorescence intensity of Alexa Fluor 647 in cells after incubation at 37°C for 1 hour, the cells treated with the peptide fluorescent conjugate 1 or 12 in which a fluoroalkyl group was linked to the side chain via a linking group all had a higher fluorescence intensity than the cells treated with the fluorescent dye 1. Specifically, it was found that the average value of the fluorescence intensities of Alexa Fluor 647 in cells treated with the peptide fluorescent conjugate 12 was about 14 times greater for the cells treated with the fluorescent dye 1. Based on these results, it was found that peptides in which a fluoroalkyl group was linked to the side chain via a linking group had higher efficiency of uptake into cells and a better ability to permeate a cell membrane than the fluorescent dye.

### [Industrial Applicability]

The present invention provides a peptide having an amino acid residue containing a fluorine atom in the side chain. Since the fluorine-containing peptide according to the present invention is highly effective at permeating a cell membrane, for example, it is expected to be used as a physiologically active substance such as a carrier for introducing medicinal components into target cells in the field of pharmaceuticals.

## Claims

1. A peptide in which 2 or more amino acids are peptide-bonded,
wherein at least one side chain of an amino acid residue constituting the peptide is represented by the following General Formula (1): 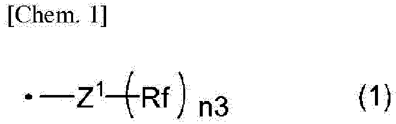 [in the formula, Z¹ is a divalent, trivalent, or tetravalent linking group other than an alkylene group; Rf is a C₁₋₃₀ alkyl group substituted with at least two fluorine atoms (the C₁₋₃₀ alkyl group may have 1 to 5 ether-bonding oxygen atoms between carbon atoms when the number of carbon atoms is 2 or more), -SF₅, or -SF₄-CR¹⁰¹R¹⁰²-CR¹⁰³R¹⁰⁴Cl (R¹⁰¹, R¹⁰², R¹⁰³, and R¹⁰⁴ are each independently a hydrogen atom, a fluorine atom, or a chlorine atom, and two or more of R¹⁰¹, R¹⁰², R¹⁰³, and R¹⁰⁴ are fluorine atoms); and n3 is 1, 2, or 3, and the black circle indicates a bonding site].

2. The peptide according to claim 1,
wherein Rf is a group represented by the following General Formula (f-1) or (f-2): 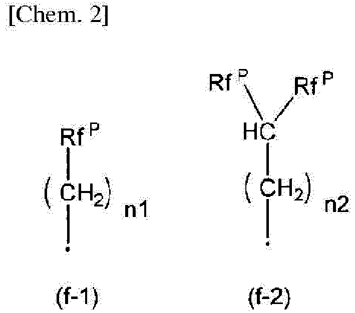 [in the formula, Rf^{P} represents a fully halogenated C₁₋₁₀ alkyl group including at least two fluorine atoms (the C₁₋₁₀ alkyl group may have an ether-bonding oxygen atom between carbon atoms when the number of carbon atoms is 2 or more), n1 is an integer of 0 to 10, n2 is an integer of 0 to 9, and the black circle indicates a bonding site].

3. The peptide according to claim 1 or 2,
wherein Z¹ is a linking group having a ring group.

4. The peptide according to claim 3,
wherein the ring group includes a 1,4-phenylene group or a 1,3,5-substituted phenyl group.

5. The peptide according to claim 1 or 2,
wherein Z¹ is a linking group having no ring group.

6. The peptide according to claim 1 or 2,
wherein Z¹ is an alkylene group, -O-, -S-, -NH-, -N(CH₃)-, -N(C₂H₅)-, -N(C₃H₇)-, a trivalent nitrogen atom, -C(=O)-, -S(=O)₂-, a cycloalkylene group, a divalent to tetravalent aryl group, a divalent to tetravalent heteroaryl group, or a combination thereof (excluding a group composed of only an alkylene group).

7. The peptide according to claim 1 or 2,
wherein Z¹ is -C(=O)-, -C(=O)-O-, -O-C(=O)-, -NH-C(=O)-O-, -O-C(=O)-NH-,-C(=O)-NH-, -NH-C(=O)-, -S-S-, -S(=O)₂-NH-, -NH-S(=O)₂-, -S(=O)₂-NH-S(=O)₂-,-C(=O)-NH-Ph- (-Ph- is a 1,4-phenylene group, 1,3-phenylene group, 1,5-phenylene group, or 1,3,5-substituted phenyl group), or a combination of any of these groups and a C₁₋₆ alkylene group.

8. The peptide according to claim 1 or 2,
wherein Z¹ is a linking group represented by the following General Formula (2): 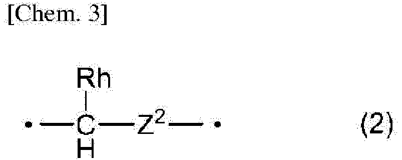 [in the formula, Z² is a divalent, trivalent, or tetravalent linking group other than an alkylene group; Rh is a hydrogen atom or a C₁₋₆ alkyl group, and the black circle indicates a bonding site].

9. The peptide according to claim 1 or 2,
wherein the amino acid residue whose side chain is a group represented by General Formula (1) is an amino acid residue in which 1 to 3 Rfs are directly or indirectly linked to a side chain of a natural amino acid.

10. The peptide according to claim 1 or 2,
wherein the C-terminal or N-terminal is optionally protected with a protecting group.

11. The peptide according to claim 1 or 2, which is a tripeptide represented by the following General Formula (11): 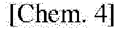 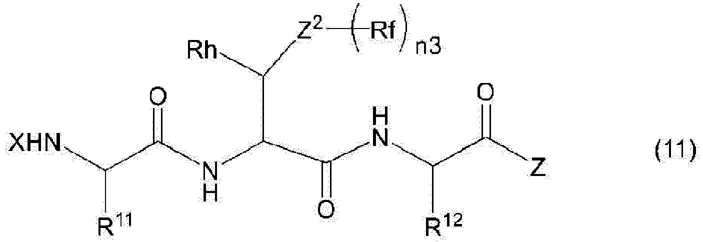 [in the formula, Rf and n3 are the same as Rf and n3 in General Formula (1); Z² is a divalent, trivalent, or tetravalent linking group other than an alkylene group; Rh is a hydrogen atom or a C₁₋₆ alkyl group; R¹¹ and R¹² are each independently a C₁₋₆ alkyl group or a benzyl group; X is a 9-fluorenylmethyloxycarbonyl group or a tert-butoxycarbonyl group; and Z is a C₁₋₆ alkoxy group, a hydroxyl group, or an amino group].

12. The peptide according to claim 1 or 2, which has an ability to permeate a cell membrane.
